# EUROPÄISCHE PATENTSCHRIFT

(11) **EP 3 275 313 B1**
(45) Veröffentlichungstag und Bekanntmachung des Hinweises auf die Patenterteilung: **14.02.2024**
(21) Anmeldenummer: 16207287.0
(22) Anmeldetag: 29.12.2016
(51) Int. Cl.: A22B 5/00, A22C 17/00, B26D 5/00, B26F 3/00, G01N 33/02

(54) **VORRICHTUNG ZUM ERFASSEN UND AUSWERTEN VON PRODUKTSPEZIFISCHEN INFORMATIONEN VON PRODUKTEN DER NAHRUNGSMITTEL VERARBEITENDEN INDUSTRIE SOWIE SYSTEM MIT EINER SOLCHEN VORRICHTUNG UND VERFAHREN ZUM VERARBEITEN VON PRODUKTEN DER NAHRUNGSMITTEL VERARBEITENDEN INDUSTRIE**
DEVICE FOR DETECTING AND EVALUATING PRODUCT-SPECIFIC INFORMATION OF PRODUCTS OF THE FOOD PROCESSING INDUSTRY, AND SYSTEM COMPRISING SUCH A DEVICE AND METHOD OF PROCESSING PRODUCTS OF THE FOOD PROCESSING INDUSTRY
DISPOSITIF POUR SAISIR ET ÉVALUER DES INFORMATIONS SPÉCIFIQUES AUX PRODUITS SUR DES PRODUITS DE L'INDUSTRIE DE TRAITEMENT DES ALIMENTS ET SYSTÈME COMPRENANT UN TEL DISPOSITIF ET PROCÉDÉ POUR TRAITER DES PRODUITS DE L'INDUSTRIE DE TRAITEMENT DES ALIMENTS

(30) Priorität: 29.07.2016 US 201662368941 P
(43) Veröffentlichungstag der Anmeldung: 31.01.2018
(73) Patentinhaber: Nordischer Maschinenbau Rud. Baader GmbH + Co. KG, 23560 Lübeck (DE); John Bean Technologies Corporation, Chicago, IL 60602 (US)
(72) Erfinder: STEFFENS, Alexander, 23566 Lübeck (DE); BLAINE, George R., Lake Stevens, WA 98258 (US); HOCKER, Jon A., Bothell, WA 98012-8860 (US)
(74) Vertreter: Stork Bamberger Patentanwälte PartmbB

(56) Entgegenhaltungen:
- EP-A1- 2 531 038
- US-A- 5 162 016
- US-A- 5 937 080
- US-A1- 2002 067 797
- US-A1- 2014 205 739
- US-B2- 8 351 672

## Beschreibung

Die Erfindung betrifft eine Vorrichtung, ausgebildet und eingerichtet zum Erfassen und Auswerten von produktspezifischen Informationen von Produkten der Nahrungsmittel verarbeitenden Industrie, wie in Anspruch 1 definiert.

Die Erfindung betrifft weiterhin ein System, ausgebildet und eingerichtet zum Verarbeiten von Produkten der Nahrungsmittel verarbeitenden Industrie, wie in Anspruch 10 definiert.

Die Erfindung ist des Weiteren auf ein Verfahren zum Verarbeiten von Produkten der Nahrungsmittel verarbeitenden Industrie gerichtet, wie in Anspruch 16 definiert.

Solche Vorrichtungen kommen insbesondere in der Nahrungsmittel verarbeitenden Industrie zum Einsatz. Bei vielen Produkten ist es für die weitere Verarbeitung an einer nachgeordneten Station, also z.B. das Sortieren, Verpacken und insbesondere das Schneiden zum Entfernen unerwünschter Bereiche und/oder zum Portionieren von Bedeutung, produktspezifische Informationen zu erfassen. Das Erfassen produktspezifischer Informationen und Daten kann u.a. das Erfassen der äußeren Kontur, der Topographie, der äußeren Abmessungen in Länge und Breite, des Gewichts, der Dicke, sowie das Erfassen von Fehlerstellen z.B. in Form von Blutstellen, Gräten, Grätenresten, Grätenbereichen und deren Position innerhalb des Produktes, sowie Gewebestruktur etc. einschließen. Das Erfassen kann durch Detektieren, Ermitteln, Scannen, Aufnehmen und dergleichen erfolgen. Bei bisher bekannten Vorrichtungen der gattungsgemäßen Art, die ausschließlich für das Erfassen der produktspezifischen Informationen eingerichtet sind, wird eine Röntgeneinheit zum Erfassen der produktspezifischen Informationen eingesetzt. Die Röntgeneinheit liefert einen ersten Datensatz an die Steuerungseinheit, z.B. einen Bildverarbeitungs-Computer (auch CPU genannt). Aus diesem Datensatz werden - im Beispielsfall einer Verarbeitung der Produkte durch Schneiden - mittels der Steuerungseinheit z.B. Schnittpfade für das jeweilige Produkt, z.B. für eine zu schneidende Grätenzone, generiert. Des Weiteren kann der von der Röntgeneinheit ermittelte und an die Steuerungseinheit weitergeleitete Datensatz dazu dienen, z.B. die äußere Kontur des zu verarbeitenden Produkts zu bestimmen. Aus der äußeren Kontur und den Schnittpfaden wird dann ein Datensatz z.B. zur Weitergabe an eine nachgeordnete Station generiert. Mit anderen Worten stellt die Steuerungseinheit z.B. ein Röntgenbild als Nachricht zur Verfügung, das die Information trägt, welche Außenkontur das Produkt aufweist und wo die Schnittpfade liegen.

Der Einsatz der Röntgeneinheit erfordert einen möglichst gleichmäßigen, strukturlosen und unterbrechungsfreien, Röntgenstrahl durchlässigen Transportförderer, um eine optimal Bildqualität zu erzielen. Insbesondere ist der Transportförderer geschlossen und in der Transportebene spaltfrei ausgebildet. Das bedeutet, dass insbesondere in der Transportebene des Transportförderers quer zur Transportrichtung T auf einen Spalt, der in gattungsfremden Vorrichtungen zur Verarbeitung von Produkten zur Aufnahme eines Schneidmittels, z.B. einer Klinge und insbesondere eines Schneidwasserstrahls, ausgebildet ist, verzichtet wird, um eine durchgängige und vollflächige Unterstützung der Produkte auf dem Transportförderer der Vorrichtung zum Erfassen und Auswerten der produktspezifischen Informationen während des Transports vom Einlaufende zum Auslaufende zu gewährleisten. Das Auswerten schließt insbesondere auch das Berechnen, Umrechnen etc. ein.

Eine Weiterverarbeitung und insbesondere ein Abgleich ("matching") des von der Vorrichtung bereitgestellten Röntgenbildes als Datensatz mit anderen optischen Systemen, wie z.B. einer optischen Kamera einer nachgeordneten Verarbeitungsstation, ist aufgrund der unterschiedlichen bildgebenden Verfahren komplex und ungenau. Ein weiteres Problem bekannter Vorrichtungen besteht darin, dass eine über hartes Gewebe, wie z.B. Gräten, Grätenreste, Sehnen etc. hinausgehende Erkennung unerwünschter Bereiche, wie z.B. Blutflecken, Fettstreifen, Parasiten in den Produkten sowie das Erkennen von Umrissen, Konturen und dergleichen von der Röntgeneinheit nicht oder nur ungenau zu erreichen ist. Anders ausgedrückt ist das Einsatzgebiet bestehender Vorrichtungen mit einer Röntgeneinheit sowie der Informationsgehalt des Röntgenbildes beschränkt.

Diese Problematik trifft insbesondere bei Systemen und Verfahren der Nahrungsmittel verarbeitenden Industrie zu, bei denen solche Vorrichtungen der genannten Art zum Einsatz kommen. Bei den zu verarbeitenden Produkten, beispielsweise Geflügelfilets oder insbesondere Fischfilets, werden mit solchen Systemen und Verfahren der eingangs genannten Art z.B. einzelne Bereiche, z.B. innenliegende Knochen, Gräten, Grätenbereiche oder dergleichen aus den Produkten herausgeschnitten. Des Weiteren können auch andere Fehlerstellen, wie z.B. Blutflecken, Fettstreifen oder dergleichen entfernt werden. Neben dem Ab- und Herausschneiden von unerwünschten Bereichen von bzw. aus dem Produkt umfasst das Verarbeiten der Produkte auch deren Portionierung. Um die Fehlerstellen, insbesondere feste Gewebebestandteile, wie z.B. Gräten und dergleichen, zu erfassen, ist eine Röntgeneinheit vorgesehen, mittels der diese Fehlerstellen innerhalb des Produktes zu identifizieren sind. Für den Betrieb der Röntgeneinheit ist es - wie weiter oben erwähnt - notwendig, einen Transportförderer vorzusehen, der ein möglichst gleichmäßiges und strukturloses Förderband verwendet, um eine optimale Bildqualität zu erzielen. Für die Verarbeitung der Produkte, nämlich insbesondere das Schneiden mittels einer Wasserstrahleinheit, ist ein strukturiertes , metallenes Gitterband als Transportförderer zweckdienlich, das den Beanspruchungen durch die Wasserstrahleinheit standhält, für die Röntgeneinheit jedoch ungeeignet ist. Die bekannten und gattungsbildenden Systeme weisen aufgrund der divergierenden Anforderungen an die Transportförderer mindestens zwei an die individuellen Erfordernisse angepasste Transportförderer auf, nämlich einen ersten für die Vorrichtung und einen anderen für die Verarbeitungsstation. Das bedeutet, dass die bekannten Systeme mindestens eine Übergabe der Produkte von einem ersten Transportförderer an einen nachfolgenden Transportförderer enthalten. Durch diese Übergabe kann es zu Veränderungen, Verschiebungen, Verwindungen etc. der Lage/Position der Produkte auf den Transportförderern und insbesondere auch zu Stauchungen und Streckungen der Produkte kommen. Trotz dieser Problematik ist sicherzustellen, dass die Produkte nach der Übergabe auf den Transportförderer der Verarbeitungsstation identifiziert und bezüglich ihrer relativen Positionen auf den Transportförderern abgeglichen ("gematcht") werden, um das Verarbeiten der Produkte, nämlich insbesondere das Schneiden der Produkte individuell und korrekt ausführen zu können. Anders ausgedrückt müssen die Schnittpfade, die zu einem Produkt auf dem ersten Transportförderer ermittelt wurden, für dasselbe Produkt auf dem zweiten Transportförderer ggf. angepasst werden, bzw. muss die Trenneinheit ggf. an die geänderte Position/Lage der Produkte und damit der Schnittpfade angepasst werden.

Dazu ist bei den bekannten Systemen und Verfahren einerseits eine Produktverfolgung ("tracking") der Produkte während des Transports auf den Transportförderern notwendig, um die Produkte zu identifizieren bzw. die von der Steuerungseinheit empfangenen Datensätze zuordnen zu können. Andererseits müssen die von der Röntgeneinheit generierten Datensätze einem Abgleich ("matching") mit den von der optischen Kamera der Verarbeitungsstation generierten Datensätze unterzogen werden. Für die bekannten Systeme und Verfahren beispielhaft sendet die Röntgenkamera oder der Detektor der Röntgeneinheit ihre Bilddaten in Bildstreifen zur Steuerungseinheit. Während des Röntgens wird jedes Produkt auf dem Transportförderer zwischen der Röntgenquelle und der Röntgenkamera hindurchgefördert. In der Steuerungseinheit werden die empfangenen Bildstreifen auf Produkte untersucht. Bei Vorhandensein eines Produktes oder Produktstückes in den Bildstreifen wird ein vollständiges Röntgenbild des gesamten Produktes durch Zusammensetzen der Bildstreifen generiert. Der Produktbereich des zusammengesetzten Röntgenbildes wird auf Gräten oder anderes Hartgewebe untersucht. Es werden in einem Speicher der Steuerungseinheit Datenstrukturen mit Koordinaten von Linien aufgebaut, die die vermeintlichen Gräten darstellen. Aufgrund der Liniendaten wird eine Grätenzone geschätzt und hieraus ein Schnittpfad berechnet. Dieser Schnittpfad wird wiederum aus Liniensegmenten zusammengesetzt gespeichert, und zwar in Koordinaten relativ zum Röntgenbild und relativ zur Position auf dem Transportförderer. In demselben Röntgenbild wird mittels der Steuerungseinheit des Weiteren die äußere Kontur des Produktes bestimmt und ebenfalls in Form von Liniensegmenten gespeichert. Aus der äußeren Kontur und dem oder jedem Schnittpfad wird eine Nachricht, also ein erster Datensatz als Übergabebild generiert. Dieser erste Datensatz wird an die Steuerungseinheit der nachgeordneten Verarbeitungsstation, die auch die Steuerungseinheit des Systems sein kann, weitergeleitet.

Nach der Übergabe des Produktes vom ersten Transportförderer auf den nachfolgenden Transportförderer der Verarbeitungsstation erfolgt das Erfassen produktspezifischer Informationen mittels einer optischen Kamera. Die erfassten Informationen bilden einen vierten Datensatz. Sobald die Verarbeitungsstation ein Produkt erkannt und aufgenommen hat, werden mittels der Steuerungseinheit alle bisher empfangenen und noch nicht verarbeiteten ersten Datensätze durchsucht. Es wird der erste Datensatz mit der höchsten Übereinstimmung ausgewählt. Zum Identifizieren der Produkte sowie zum Berechnen der relativen Produktpositionen jedes Produktes auf den beiden Transportförderern werden der vierte Datensatz und der erste Datensatz abgeglichen und ausgewertet. Die Nummerierung der Datensätze hat keine logische oder inhaltliche Bedeutung sondern dient einzig dem Zweck, die Datensätze bezüglich ihres Ursprungs zu kennzeichnen.

Die bekannten Systeme und Verfahren weisen den Nachteil auf, das das Identifizieren sowie das Abgleichen und das Auswerten von Datensätzen auf der Basis unterschiedlicher Bildgebungsverfahren, nämlich dem Röntgenbild (erster Datensatz) der Vorrichtung einerseits und einem optischen Bild (vierter Datensatz) der Verarbeitungsstation andererseits, erfolgt. Anders ausgedrückt erfolgt das "matching", indem ein optisches Bild der optischen Kamera der Verarbeitungsstation mit einem Röntgenbild der Röntgenkamera der Vorrichtung verglichen wird. Zum einen ist dieser "matching"-Prozess aufgrund der unterschiedlichen Bildgebungsverfahren sehr komplex. Zum anderen fehlt es bei diesem Abgleich an der notwendigen Präzision. Anders ausgedrückt fehlt es bei dem bekannten "matching" an der gewünschten und erforderlichen Genauigkeit. Ein weiterer Nachteil besteht darin, dass mittels der Röntgeneinheit nicht alle unerwünschten Bereiche, die es zu schneiden gilt, zu erkennen bzw. nur unzureichend zu erkennen sind. Mit anderen Worten ist eine über Gräten, Grätenbereiche und anderes Hartgewebe hinausgehende Erkennung von Fehlerstellen durch die Röntgeneinheit schwierig. Ein weiterer Nachteil besteht darin, dass die Produkte während des Transports durch das System verfolgt ("getrackt") werden müssen, was zum einen aufwendig und zum anderen zu ungenauen Ergebnissen führt.

Das Dokument US-B2-8.351.672 offenbart den Oberbegriff von Anspruch 1.

Der Erfindung liegt somit die Aufgabe zugrunde, eine einfache Vorrichtung zu schaffen, mittels der ein Datensatz mit höherem und präziserem Informationsgehalt generierbar ist. Die Aufgabe besteht weiterhin darin, ein verbessertes System zum Verarbeiten von Produkten zu schaffen, das ein einfacheres und präziseres Identifizieren sowie Abgleichen und Auswerten der empfangenen Datensätze ermöglicht. Die Aufgabe besteht auch darin, ein entsprechendes Verfahren vorzuschlagen.

Diese Aufgabe wird durch eine Vorrichtung mit den eingangs genannten Merkmalen dadurch gelöst, dass demselben Transportförderer zwischen dessen Einlaufende und Auslaufende mindestens eine optische Kamera zugeordnet ist, mittels der zusätzlich zu der Röntgeneinheit produktspezifische Informationen von den auf dem Transportförderer transportierten Produkten erfassbar sind, wobei die optische Kamera mit einer Steuerungseinheit verbunden ist, die zum Empfangen und Auswerten der von der optischen Kamera erfassten, produktspezifischen Informationen, die einen zweiten Datensatz bilden, ausgebildet und eingerichtet ist. Durch diese erfindungsgemäße Ausführung ist die Vorrichtung in der Lage, präzisere und vor allem aussagekräftigere Informationen für eine potentielle weitere Verarbeitung bereitzustellen. Durch die Röntgeneinheit einerseits und mindestens eine optische Kamera andererseits können mehr und vor allem auch genauere Informationen generiert werden, die in Form von einzelnen, separaten Datensätzen oder in Form eines aus den einzelnen Datensätzen miteinander verknüpften Datensatzes verarbeitbar sind. Mit der erfindungsgemäßen Vorrichtung wird eine exaktere und präzisere Bildregistrierung möglich, die insbesondere auch ungleichmäßig verteilte Stauchungen/Streckungen innerhalb der Produkte besser abbildet.

Vorzugsweise ist mindestens eine optische Kamera oberhalb des Transportförderers angeordnet. Das bedeutet, dass z.B. das Produkt ausleuchtende Lichtstrahlen von oben in einem Winkel auf den Transportförderer bzw. auf das auf dem Transportförderer liegende Produkt treffen, so dass die Kamera die reflektierten Lichtstrahlen aufnehmen kann. Einfach ausgedrückt beinhaltet der Begriff "oberhalb", dass die Kamera und vorzugsweise mindestens eine Lichtquelle schräg oder senkrecht von oben auf das Produkt gerichtet sind. Besonders vorteilhaft ist eine Anordnung, bei der die optische Kamera senkrecht oberhalb des Transportförderers angeordnet ist, so dass mittels der optischen Kamera eine Draufsicht auf das Produkt gewährleistet ist. Selbstverständlich ist auch eine Anordnung unterhalb des Transportförderers möglich.

Eine bevorzugte Weiterbildung der Vorrichtung ist dadurch gekennzeichnet, dass dem Transportförderer mindestens zwei optische Kameras zugeordnet sind, die mit einem unterschiedlichen Bildgebungsverfahren ausgestattet sind. Das besonders vorteilhafte an dieser Ausführungsform besteht darin, dass z.B. eine einfache optische Kamera, beispielsweise eine Grauwertkamera, zur Aufnahme eines vollständiges Bildes (Foto) des Produktes für die Bildregistrierung, z.B. die äußere Kontur (als produktspezifische Information) sowie eine Spezialkamera, beispielsweise eine Multispektralkamera, zur Aufnahme von unerwünschten Bereichen (als produktspezifische Information), wie z.B. Blutflecken, Fettstreifen oder dergleichen, einsetzbar ist.

Vorteilhafterweise ist entsprechend mindestens eine optische Kamera eine Multi- oder Hyperspektralkamera. Der Einsatz einer solchen Kamera ermöglicht es, zusätzlich zu den produktspezifischen Informationen, die durch die Röntgeneinheit erfassbar sind, weitere und insbesondere von der Röntgeneinheit nicht oder nur unzureichend erfassbare unerwünschte Bereiche zu erfassen, um die Qualität der zu erfassenden produktspezifischen Informationen zu optimieren.

Vorzugsweise ist entsprechend mindestens eine optische Kamera als Grauwertkamera und/oder RGB-Kamera und/oder IR- und/oder UV-Kamera ausgebildet ist. Mit dieser bevorzugten Ausgestaltung ist die Bandbereite der zu erfassenden produktspezifischen Informationen wesentlich verbessert und ein potentieller Abgleich mit optischen Bildern optischer Kameras von nachgeordneten Stationen vereinfacht.

Vorzugsweise ist der Transportförderer ein umlaufend angetriebenes und auf der Transportfläche strukturarmes Röntgenförderband, das aus Kunststoff hergestellt ist, mit einem Transportband als Obertrum und einem Rückführband als Untertrum. Die Produkte liegen auf der Transportfläche auf. Diese Transportfläche ist einerseits so eben, also insbesondere ohne Durchbrüche, Öffnungen oder dergleichen ausgebildet, dass Röntgenaufnahmen störungsfrei aufzunehmen sind. Anderseits weist die Transportfläche eine leichte, im Millimeterbereich liegende Oberflächenrauigkeit auf, um ein (Ver-)Rutschen der Produkte auf dem Transportförderer zu verhindern. Entsprechend ist die Transportfläche strukturarm ausgebildet. Wie bereits weiter oben erwähnt, weist der Transportförderer eine in der Transportebene spaltfreie Transportfläche auf. Das bedeutet, dass die Produkte auf der Transportfläche jederzeit und an jedem Ort vollflächig unterstützt werden. Anders ausgedrückt bildet der Transportförderer mit seiner Transportfläche des Transportbandes eine durchgängige Auflagefläche für die Produkte. Dadurch kann eine optimierte Röntgenaufnahme gewährleistet werden.

Eine bevorzugte Weiterbildung ist dadurch gekennzeichnet, dass der Transportförderer an eine Steuerungseinheit angeschlossen ist, die zum Empfangen und Auswerten von Bewegungsdaten des Transportförderers ausgebildet und eingerichtet ist. Durch die Anbindung des Transportförderers an die Steuerungseinheit ist die Zuordnung der einzelnen Produkte vereinfacht. Anders ausgedrückt ermöglicht die beanspruchte Lösung eine verbesserte und präzisere Zuordnung der durch die Röntgeneinheit und jede optische Kamera erfassten produktspezifischen Informationen zu einem Produkt. Da die Röntgenkamera und die optische Kamera oberhalb desselben Transportförderers und entsprechend dicht nebeneinander liegen, können die Bildinformationen, also die Datensätze ohne jeden Abgleich ("matching") direkt auf Basis der Zeitdifferenz, die von der Geschwindigkeit des Transportförderers abhängig ist, übereinander gelegt werden.

In einer bevorzugten Ausführungsform ist die Röntgenquelle oberhalb des Transportbandes und die Röntgenkamera zwischen dem Transportband und dem Rückführband angeordnet. Damit ist ein besonders deutliches und aussagekräftiges Röntgenbild erfassbar, wodurch die Erkennung bzw. Erfassung der produktspezifischen Informationen, beispielsweise die Lage und Position von Gräten, Grätenbereichen und dergleichen innerhalb des Produktes verbessert wird.

Die Erfindung ist dadurch gekennzeichnet, dass die Röntgeneinheit und jede optische Kamera sowie der Transportförderer an eine Steuerungseinheit zum Empfangen und Auswerten der Datensätze angeschlossen sind, wobei die Steuerungseinheit mit mindestens einem Prozessor ausgestattet ist, der zum Integrieren/Implementieren mindestens von Teilen der durch die Röntgeneinheit erfassten produktspezifischen Informationen eines Produktes in das optische Bild der optischen Kamera zum selben Produkt zur Bildung eines optischen Übergabebildes, das einen dritten Datensatz bildet, konfiguriert ist. Die von der Röntgeneinheit und jeder optischen Kamera erzeugten Bilder bzw. Bilddaten werden von der Steuerungseinheit als Datensätze empfangen und ausgewertet. Besonders vorteilhaft wird der erste Datensatz (Ergebnis der Röntgeneinheit) in den zweiten Datensatz (Ergebnis der optischen Kamera) integriert/implementiert, so dass ein dritter Datensatz gebildet wird. Dieser dritte Datensatz ist ein optisches Übergabebild. Besonders bevorzugt wird die Lage/Position/Ausdehnung von Gräten, Grätenbereichen und anderen Hartgewebeteilen (als produktspezifische Informationen, die von der Röntgeneinheit erfasst wurden = erster Datensatz) über das optische Bild (= zweiter Datensatz), das z.B. das gesamte Produkt oder die äußere Kontur desselben abbildet, gelegt, so dass im Ergebnis ein optisches Bild (= dritter Datensatz), nämlich das Übergabebild, entsteht. Dieses optische Bild als dritter Datensatz liefert entsprechend Informationen zur Form/Gestalt des Produktes z.B. durch die äußere Kontur, sowie zu Fehlerstellen, z.B. Grätenbereiche etc., die entfernt werden sollen. Diese Ausführungsform erleichtert und vereinfacht eine mögliche Weiterverarbeitung der produktspezifischen Informationen. Dadurch, dass die Röntgeneinheit und die oder jede optische Kamera der Vorrichtung einem einzigen Transportförderer zugeordnet sind, kann auf einen Abgleich der Bilder, also der Röntgenbilder und der Bilder optischer Kameras, verzichtet werden. Mit anderen Worten ist die Zuordnung der produktspezifischen Informationen zu einem Produkt einfach und präzise gewährleistet, insbesondere dann, wenn die Position der einzelnen Produkte anhand der Bewegungsdaten des Transportförderers bestimmbar ist. Die zusätzliche optische Kamera kann optional auch Informationen zu Fehlerstellen liefern, die von der Steuerungseinheit erfasst und ausgewertet werden können.

Besonders bevorzugt ist die genannte Weiterbildung dadurch gekennzeichnet, dass die Steuerungseinheit mit dem Prozessor zum Bestimmen von Schnittpfaden aus den von der optischen Kamera erfassten produktspezifischen Informationen und aus den von der Röntgeneinheit erfassten produktspezifischen Informationen konfiguriert ist, wobei die Schnittpfade aus den von der Röntgeneinheit erfassten produktspezifischen Informationen eines Produktes direkt über das optische Bild der optischen Kamera zum selben Produkt gelegt werden, derart, dass die Steuerungseinheit ein optisches Bild einer optischen Kamera, nämlich das Übergabebild, zur Weiterverarbeitung bereitstellt. Für den Fall, dass neben der Röntgeneinheit nur eine einzelne optische Kamera vorgesehen ist, kann die Röntgeneinheit mittels der Steuerungseinheit beispielsweise ausschließlich Schnittpfade zur Verfügung stellen, während die optische Kamera mittels der Steuerungseinheit das gesamte Produkt oder die äußere Kontur und ggf. zusätzlich Schnittpfade, welche die Schnittpfade aus dem Röntgenbild qualitativ ergänzen, zur Verfügung stellt. Diese Informationen (Schnittpfade und Produktfoto und/oder äußere Kontur) können dann zu einem gemeinsamen dritten Datensatz miteinander kombiniert werden, und zwar auf der Basis eines optischen Bildes. Für den Fall, dass neben der Röntgeneinheit zwei optische Kameras vorgesehen sind, kann die Röntgeneinheit mittels der Steuerungseinheit beispielsweise ausschließlich Schnittpfade zur Verfügung stellen, während die erste optische Kamera mittels der Steuerungseinheit ein Produktfoto und/oder die äußere Kontur und die zweite optische Kamera mittels der Steuerungseinheit Schnittpfade zur Verfügung stellt.

Die erfindungsgemäße Vorrichtung ist als Stand-alone-Lösung einsetzbar. Bevorzugt ist die Vorrichtung jedoch mit einer nachgeordneten Verarbeitungsstation gekoppelt.

Das bedeutet, dass die aus der zuvor beschriebenen Vorrichtung gewonnenen Erkenntnisse, Daten, Informationen etc. zur weiteren Verarbeitung in der Verarbeitungsstation einsetzbar sind. Diese weitere Verarbeitung kann das Sortieren, das Verpacken und insbesondere das Schneiden zu Zwecken des Entfernens unerwünschter Bereiche und/oder des Portionierens umfassen.

Die Aufgabe wird entsprechend auch durch ein System mit den eingangs genannten Merkmalen dadurch gelöst, dass die Vorrichtung, ausgebildet und eingerichtet zum Erfassen und Auswerten von produktspezifischen Informationen der Produkte, nach einem der hierzu genannten Ansprüche ausgebildet ist. Die sich daraus ergebenden Vorteile wurden bereits im Zusammenhang mit der Vorrichtung beschrieben, weshalb zur Vermeidung von Wiederholungen auf die entsprechenden Passagen verwiesen wird. Besonders vorteilhaft ist die Tatsache, dass zum Identifizieren der Produkte sowie Abgleichen und Auswerten optische Bilder von optischen Kameras übereinandergelegt werden können. Anders ausgedrückt erfolgt das "matching" zwischen optischen Bildern mit gleichen oder zumindest ähnlichen Bildgebungsverfahren, so dass die Qualität des "matching"-Prozesses vereinfacht und verbessert ist.

Eine besonders bevorzugte Weiterbildung ist entsprechend dadurch gekennzeichnet, dass sämtliche Steuerungseinheiten des Systems miteinander in Wirkverbindung stehen und mindestens eine Steuerungseinheit einen Prozessor umfasst, der konfiguriert ist, optische Bilder der optischen Kameras der Vorrichtung einerseits und der Verarbeitungsstation andererseits übereinanderzulegen, derart, dass das Identifizieren der Produkte sowie das Abgleichen der relativen Positionen jedes Produktes auf den beiden Transportförderern der Vorrichtung einerseits und der Verarbeitungsstation andererseits ausschließlich anhand optischer Bilder optischer Kameras erfolgt.

In einer besonders vorteilhaften Weiterbildung sind die Vorrichtung und die Verarbeitungsstation an eine Steuerungseinheit angeschlossen, die einen Prozessor umfasst, der konfiguriert ist, ein optisches Übergabebild, das aus einem optischen Bild einer optischen Kamera der Vorrichtung, angereichert um von der Röntgeneinheit der Vorrichtung erfasste produktspezifische Daten, gebildet ist, und ein optisches Bild der optischen Kamera der Verarbeitungsstation übereinanderzulegen, derart, dass die Trenneinheit mittels der Steuerungseinheit für jedes Produkt auf der Basis individuell ermittelter Schnittpfade steuerbar ist. Mit anderen Worten werden der dritte Datensatz und der vierte Datensatz zum "matching" verwendet.

Bevorzugt umfasst die Trenneinheit eine Wasserstrahleinheit mit mindestens einer Düse. Mit dieser Ausbildung können besonders schnell und präzise individuelle Trenn- und Trimmschnitte zum Entfernen unerwünschter Bereiche und/oder zum Portionieren ausgeführt werden.

Besonders bevorzugt ist mindestens je eine optische Kamera in der Vorrichtung einerseits und der Verarbeitungsstation andererseits mit dem gleichen Bildgebungsverfahren ausgestattet. Dadurch wird das "matching" weiter vereinfacht. Vorteilhafterweise umfasst mindestens eine Steuerungseinheit mindestens einen Speicher, mindestens eine Eingabeeinrichtung sowie mindestens eine Ausgabeeinrichtung.

Die Aufgabe wird auch durch ein Verfahren mit den eingangs genannten Verfahrensschritten gelöst, indem auf dem ersten Transportförderer zusätzlich zu den von der Röntgeneinheit erfassten produktspezifischen Informationen produktspezifische Informationen zu jedem Produkt mittels einer optischen Kamera erfasst werden, wobei die durch die dem ersten Transportförderer zugeordnete optische Kamera erfassten Informationen, die einen zweiten Datensatz bilden, von einer Steuerungseinheit empfangen und ausgewertet werden, und dass das Identifizieren der Produkte und das Abgleichen und das Auswerten der Datensätze auf der Basis der beiden von den optischen Kameras erzeugten Datensätzen, nämlich dem zweiten Datensatz und dem vierten Datensatz, erfolgt. Die sich daraus ergebenden Vorteile wurden bereits im Zusammenhang mit dem System beschrieben, weshalb zur Vermeidung von Wiederholungen auf die entsprechenden Passagen verwiesen wird.

Das Verfahren ist dadurch gekennzeichnet, dass der erste Datensatz, der die durch die Röntgeneinheit generierten Schnittpfade beinhaltet, in den zweiten Datensatz, nämlich in das durch die dem ersten Transportförderer zugeordnete optische Kamera generierte optische Bild integriert/implementiert wird, so dass ein optisches Übergabebild als dritter Datensatz zum Abgleichen und Auswerten an die Steuerungseinheit weitergegeben wird, wobei das Identifizieren der Produkte und das Abgleichen und das Auswerten der Datensätze auf der Basis des dritten und des vierten Datensatzes erfolgt.

Vorzugsweise werden die Positionen der durch die Röntgeneinheit generierten, unerwünschten Bereiche als Vektordaten, Pixeldaten oder dergleichen in das optische Bild der dem ersten Transportförderer zugeordneten optischen Kamera integriert/implementiert, und das Abgleichen ("matching") und Auswerten wird aufgrund des optischen Bildes der dem ersten Transportförderer zugeordneten optischen Kamera und des optischen Bildes der dem zweiten Transportförderer zugeordneten optischen Kamera ausgeführt. Optional können auch die von einer weiteren optischen Kamera der Vorrichtung generierten, unerwünschten Bereiche als Vektordaten, Pixeldaten oder dergleichen in das optische Bild, das zum "matching" verwendet wird, integriert/implementiert werden.

Vorteilhafterweise wird zum Identifizieren der Produkte jedes mit der dem nachfolgenden Transportförderer zugeordneten optischen Kamera aufgenommene optische Bild mit den in einem Speicher einer Steuerungseinheit gespeicherten optischen Bildern der dem ersten Transportförderer zugeordneten optischen Kamera verglichen und das Bild mit den größten Übereinstimmungen ausgewählt.

Besonders bevorzugt wird das Verfahren mit einem System nach einem der hierzu genannten Ansprüche ausgeführt.

Weitere zweckmäßige und/oder vorteilhafte Merkmale und Weiterbildungen sowie bevorzugte Verfahrensschritte ergeben sich aus den Unteransprüchen und der Beschreibung. Besonders bevorzugte Ausführungsformen der Vorrichtung und des Systems sowie das Verfahren werden anhand der beigefügten Zeichnung näher erläutert. In der Zeichnung zeigt:
- Fig. 1: eine schematische Darstellung einer ersten Ausführungsform der erfindungsgemäßen Vorrichtung mit einer Röntgeneinheit und einer optischen Kamera,
- Fig. 2: eine schematische Darstellung einer weiteren Ausführungsform der erfindungsgemäßen Vorrichtung mit einer Röntgeneinheit und zwei optischen Kameras,
- Fig. 3: eine schematische Darstellung einer ersten Ausführungsform des erfindungsgemäßen Systems,
- Fig. 4: eine schematische Darstellung einer weiteren Ausführungsform des erfindungsgemäßen Systems, und
- Fig. 5: eine schematische Darstellung einer weiteren Ausführungsform des erfindungsgemäßen Systems.

Die in der Zeichnung dargestellte Vorrichtung ist zum Erfassen und Auswerten von produktspezifischen Informationen von Fischfilets eingerichtet. Entsprechend ist das dargestellte System zum Verarbeiten, nämlich Schneiden von Fischfilets eingerichtet. Die Vorrichtung und das System sind jedoch in gleicher Weise auch für das Erfassen und Auswerten mit produktspezifischer Informationen sowie das Verarbeiten von anderen Produkten der Nahrungsmittel verarbeitenden Industrie, wie z.B. Fleisch, Geflügel eingerichtet.

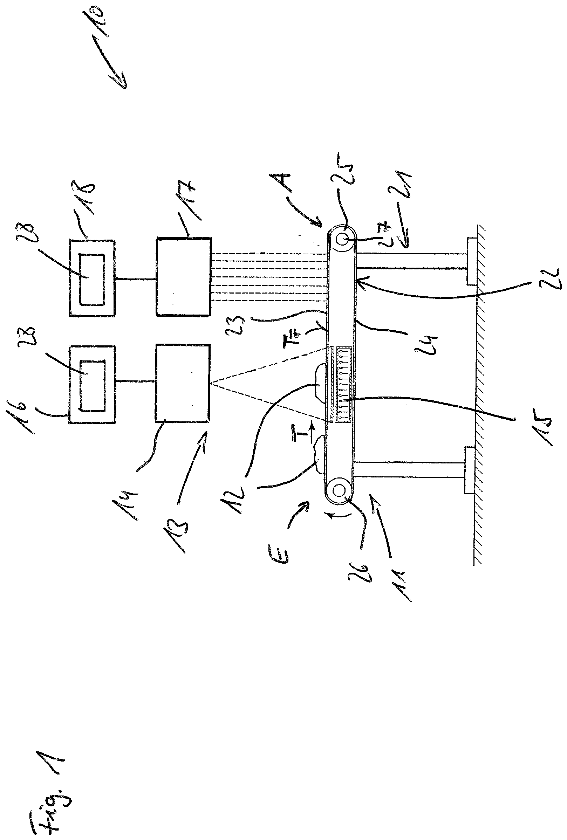

In der Figur 1 ist eine Vorrichtung 10 dargestellt, die zum Erfassen und Auswerten von produktspezifischen Informationen ausgebildet und eingerichtet ist, und die einen in der Transportebene spaltfreien Transportförderer 11 zum Transportieren vereinzelter Produkte 12 in Transportrichtung T von einem Einlaufende E zu einem Auslaufende A sowie eine Röntgeneinheit 13 mit mindestens einer Röntgenquelle 14 und mindestens einer Röntgenkamera 15 oder mindestens einem Röntgendetektor zum Erfassen der produktspezifischen Informationen umfasst, wobei Röntgenquelle 14 und Röntgenkamera 15 dem Transportförderer 11 derart zugeordnet sind, dass die Produkte 12 zwischen der Röntgenquelle 14 und der Röntgenkamera 15 entlang führbar sind. Des Weiteren umfasst die Vorrichtung 10 eine Steuerungseinheit 16, die mit der Röntgeneinheit 13 verbunden und zum Empfangen und Auswerten der von der Röntgeneinheit 13 erfassten, produktspezifischen Informationen, die einen ersten Datensatz bilden, ausgebildet und eingerichtet ist. Die Röntgeneinheit 13 liefert demnach Informationen/Daten an die Steuerungseinheit 16.

Mittels der Steuerungseinheit 16 werden die von der Röntgeneinheit 13 erfassten produktspezifischen Informationen bzw. Teile davon, wie z.B. Lage/Position von Gräten, Grätenbereiche ausgewertet und als Datensatz zur Verfügung gestellt. Die Verbindung zwischen der Röntgeneinheit 13 und der Steuerungseinheit 16 kann auf unterschiedliche Weise realisiert sein, nämlich z.B. draht-/leitungsgebunden oder leitungslos, z.B. über Funk- oder Bluetooth-Schnittstellen oder dergleichen.

Diese Vorrichtung 10 zeichnet sich erfindungsgemäß dadurch aus, dass demselben Transportförderer 11 zwischen dessen Einlaufende E und Auslaufende A mindestens eine optische Kamera 17 zugeordnet ist, mittels der zusätzlich zu der Röntgeneinheit 13 produktspezifische Informationen von den auf dem Transportförderer 11 transportierten Produkten 12 erfassbar sind, wobei die optische Kamera 17 mit einer Steuerungseinheit 18 verbunden ist, die zum Empfangen und Auswerten der von der optischen Kamera 17 erfassten, produktspezifischen Informationen, die einen zweiten Datensatz bilden, ausgebildet und eingerichtet ist.

Mittels der Steuerungseinheit 18 werden die von der optischen Kamera 17 erfassten produktspezifischen Informationen, wie z.B. Größe und Form des Produktes, dessen äußere Kontur, Länge, Breite, Dicke, Profil, Gewicht u.a., aber auch Fehlerstellen, wie z.B. Blutflecken, Fettstreifen u.a. oder Teile davon ausgewertet und als zweiter Datensatz zur Verfügung gestellt. Die Verbindung zwischen der optischen Kamera 17 und der Steuerungseinheit 18 kann auf unterschiedliche Weise realisiert sein, nämlich z.B. draht-/leitungsgebunden oder leitungslos, z.B. über Funk- oder Bluetooth-Schnittstellen oder dergleichen.

Die im Folgenden beschriebenen Merkmale und Weiterbildungen sowie Verfahrensschritte stellen für sich betrachtet oder in Kombination miteinander bevorzugte Ausführungsformen dar. Es wird ausdrücklich darauf hingewiesen, dass Merkmale und Verfahrensschritte, die in den Ansprüchen und/oder der Beschreibung und/oder der Zeichnung zusammengefasst oder in einer gemeinsamen Ausführungsform beschrieben sind, auch funktional eigenständig die weiter oben beschriebene Vorrichtung sowie das weiter unten beschriebene System und Verfahren weiterbilden können.

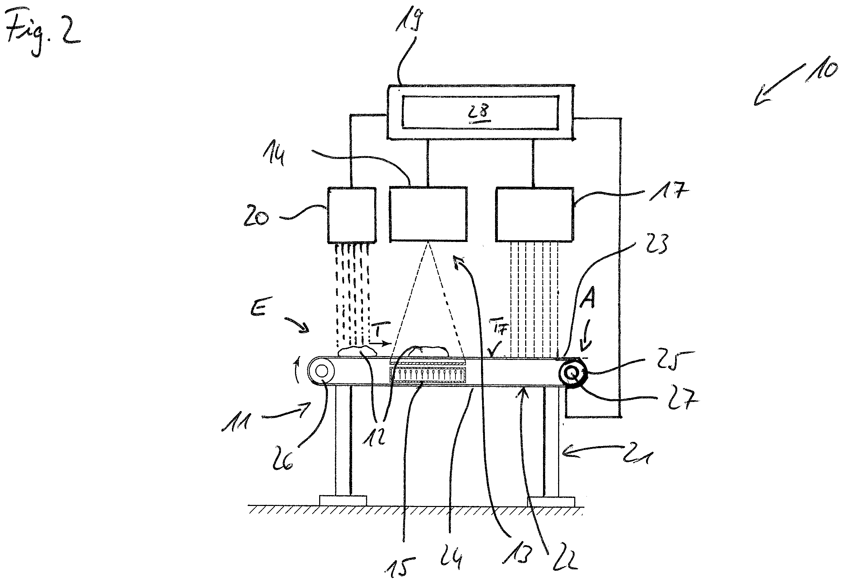

Die optische Kamera 17 kann in Transportrichtung T der Produkte 12 vor der Röntgeneinheit 13, oder wie in der Figur 1 dargestellt, hinter der Röntgeneinheit 13 angeordnet sein. Die Röntgeneinheit 13 und die optische Kamera 17 können jeweils an eine separate Steuerungseinheit 16, 18 angeschlossen sein. In anderen Ausführungsformen, wie z.B. gemäß Figur 2, können die Röntgeneinheit 13 und die optische Kamera 17 auch mit einer gemeinsamen Steuerungseinheit 19 verbunden sein.

Die Position der Röntgeneinheit 13 und/oder optischer Kamera 17 in Bezug auf den Transportförderer 11 kann variieren. Neben einer Anordnung von Röntgeneinheit 13 und/oder optischer Kamera 17 unterhalb des Transportförderers 11 ist eine Anordnung oberhalb des Transportförderers 11 bevorzugt. In der Figur 1 ist eine solche Ausführungsform beispielhaft dargestellt, bei der nicht nur die Röntgeneinheit 13 oberhalb des Transportförderers 11 angeordnet ist, sondern auch die optischen Kamera 17. Oberhalb bedeutet in diesem Zusammenhang, dass die Röntgenquelle 14 und die Lichtquelle der optischen Kamera 17 von oben auf das Produkt 12 treffen. Das kann in einem schrägen Winkel und bevorzugt senkrecht von oben erfolgen, um eine Draufsicht auf das Produkt 12 zu erhalten.

In einer bevorzugten Ausführungsform, wie sie in Figur 2 dargestellt ist, sind dem Transportförderer 11 mindestens zwei optische Kameras 17, 20 zugeordnet. Die beiden optischen Kameras 17, 20 können mit dem gleichen Bildgebungsverfahren ausgestattet sein. Vorzugsweise sind die beiden optischen Kameras 17, 20 jedoch mit einem unterschiedlichen Bildgebungsverfahren ausgestattet. Optische Kameras im Sinne der Erfindung können Grauwertkameras/-sensoren, RGB-Kameras/-sensoren sowie Infrarot- oder Ultraviolettkameras/-sensoren sein. Jede optische Kamera kann z.B. als Flächen- oder Zeilenkamera ausgeführt sein. Bei den Grauwertkameras bzw. - sensoren werden mittels fotoaktiver Bauelemente, wie z.B. Fotodiode, Fototransistor, elektromagnetische Wellen im sichtbaren Bereich in elektrische Signale auf einem Sensor gewandelt. Bei den RGB-Kameras bzw. -Sensoren wird das Licht durch Vorfilterung der eintreffenden Lichtwellen vor den Pixeln ortsabhängig in einen Rot-Grün-Blau-Kanal aufgeteilt. Bei den Infrarot- oder Ultraviolettkameras bzw. -Sensoren wird durch die Auswahl der fotoaktiven Elemente die Empfindlichkeit in den Bereich der längeren Wellenlängen (IR) oder kürzeren Wellenlängen (UV) im Vergleich zum sichtbaren Licht verschoben.

Mindestens eine der optischen Kameras 17, 20 ist eine Multi- oder Hyperspektralkamera. Mindestens eine der optischen Kameras 17, 20 ist als Grauwertkamera und/oder RGB-Kamera und/oder IR- und/oder UV-Kamera ausgebildet. In der Ausführungsform gemäß Figur 2 ist eine der optischen Kameras, beispielsweise die optische Kamera 17, eine einfache Kamera, nämlich z.B. eine Grauwertkamera. Mittels dieser optischen Kamera 17 kann z.B. die äußere Kontur als produktspezifische Information erfasst und an die Steuerungseinheit 19 weitergeleitet werden. Die andere optische Kamera 20 kann eine komplexe Kamera, nämlich z.B. eine Hyperspektralkamera sein. Mittels dieser optischen Kamera 20 können unerwünschte Bereiche, wie z.B. Fehlerstellen in Form von Blutflecken, Knochen(-reste), Knorpel, Fett, aber auch Fremdkörper, wie z.B. Glas, Kunststoff etc. erfasst und als produktspezifische Information an die Steuerungseinheit 19 weitergeleitet werden. Die Anzahl der Kameras/Sensoren und deren Positionierung entlang des Transportförderers 11 können variieren.

Der Transportförderer 11 der Vorrichtung 10 weist ein Gestell 21 zum Stützen eines Transportförderbandes auf. Das Transportförderband ist bevorzugt ein umlaufend angetriebenes und auf der Transportfläche T_{F} strukturarmes Röntgenförderband 22, das aus Kunststoff hergestellt ist, mit einem Transportband 23 als Obertrum und einem Rückführband 24 als Untertrum. Das Röntgenförderband 22 mit seiner nach oben weisenden Transportfläche T_{F} ist frei von Öffnungen, Durchbrüchen, Spalten oder dergleichen. Anders ausgedrückt ist die Transportfläche T_{F} über die gesamte Länge und Breite geschlossen ausgebildet. Das vorzugsweise endlos ausgebildete Röntgenförderband 22, das aus Gummi, Plastik oder einem anderen Kunststoff besteht und für Röntgenstrahlen durchlässig ist, ist um mindestens zwei Umlenkelemente 25, 26 gelenkt, von denen ein Umlenkelement 25 oder 26 als Antriebsrolle und das andere Umlenkelement 26 oder 25 als Umlenkrolle ausgebildet ist. Insbesondere im Bereich der Antriebsrolle, die mittels eines Antriebsmittels antreibbar ist, kann optional ein Encoder 27 vorgesehen sein, mittels dem die Position des Röntgenförderbandes 22 entlang der Länge des Transportförderers 11 und damit die Position des Produktes 12 auf dem Transportförderer 11 bestimmt bzw. überwacht werden kann. Die den Produkten 12 zugewandte Transportfläche T_{F} des Röntgenförderbandes 22 ist strukturarm ausgebildet. Das bedeutet, dass die geschlossene Transportfläche T_{F} leicht angeraut ist. Es sind aber auch vollständig glatte Transportflächen einsatzbar.

In den in der Zeichnung dargestellten Ausführungsformen ist die Röntgenquelle 14 oberhalb des Transportförderers 11 und oberhalb des Transportbandes 23 angeordnet. Die Röntgenkamera 15 ist zwischen dem Transportband 23 und dem Rückführband 24 angeordnet. Optional kann die Röntgenkamera 15 auch unterhalb des Rückführbandes 24 angeordnet sein. Andere Konstellationen von Röntgenquelle 14 und Röntgenkamera 15 in Bezug auf das Transportband 23 bzw. Rückführband 24 sind ebenfalls möglich.

Optional ist der Transportförderer 11 an eine Steuerungseinheit 19 angeschlossen, die zum Empfangen und Auswerten von Bewegungsdaten des Transportförderers 11 ausgebildet und eingerichtet ist. In der Ausführungsform gemäß Figur 2 ist der Transportförderer 11 bzw. der Encoder 27 an die gemeinsame Steuerungseinheit 19 angeschlossen, an der auch die Röntgeneinheit 13 und die optischen Kameras 17, 20 angeschlossen sind. Eine Anbindung an eine separate Steuerungseinheit, die mit den anderen Steuerungseinheiten 16, 18 in Wirkverbindung steht, ist aber ebenfalls möglich.

Eine oder jede Steuerungseinheit 16, 18, 19 umfasst mindestens einen Prozessor (CPU) 28. Bevorzugt sind die Röntgeneinheit 13 und jede optische Kamera 17, 20 sowie der Transportförderer 11 an eine gemeinsame bzw. übergeordnete Steuerungseinheit 19 zum Empfangen und Auswerten der Datensätze angeschlossen, wobei die Steuerungseinheit 19 mit mindestens einem Prozessor (CPU) 28 ausgestattet ist, der zum Integrieren/Implementieren mindestens von Teilen der durch die Röntgeneinheit 13 erfassten produktspezifischen Informationen eines Produktes in das optische Bild der optischen Kamera 17, 20 zum selben Produkt zur Bildung eines optischen Übergabebildes, das einen dritten Datensatz bildet, konfiguriert ist. Diese bevorzugte Ausführungsform ist in der Figur 2 dargestellt. In anderen Ausführungsbeispielen können auch nur die Röntgeneinheit 13 und/oder einzelne optische Kameras 17, 20 und/oder der Transportförderer 11 an die gemeinsame Steuerungseinheit 19 angeschlossen sein. Die Steuerungseinheit 19 und ggf. auch jede andere Steuerungseinheit 16, 18 umfasst neben dem mindestens einen Prozessor 28 optional einen Speicher, eine Eingabeeinrichtung, eine Ausgabeeinrichtung sowie eine Schnittstelle. Des Weiteren besteht die Möglichkeit, dass die Vorrichtung 10 über die Steuerungseinheit 19 mit einem Netzwerk, das intern (Intranet) oder extern (Internet) vernetzt sein kann, verbunden ist. Die Röntgeneinheit 13, die optischen Kameras 17, 20 sowie der Transportförderer 11 können auch an separate Steuerungseinheiten 16, 18 angeschlossen sein, die wiederum mit einer übergeordneten Steuerungseinheit zusammenwirken.

Alle Steuerungseinheiten 16, 18, 19 und insbesondere die Steuerungseinheit 19, also eine Industriesteuerung, ein Computer oder dergleichen, umfasst wie erwähnt mindestens einen Prozessor 28 (CPU), der zum Aufnehmen der Datensätze von der Röntgeneinheit 13 und jeder optischen Kamera 17, 20 ausgebildet und eingerichtet ist. Vorzugsweise ausgewählte produktspezifische Informationen, die von der Röntgeneinheit 13 erfasst wurden, beispielsweise die Lage einer Reihe von Pinbones in einem Fischfilet (z.B. erster Datensatz), werden mittels des Prozessors 28 in das optische Bild der optischen Kamera 17, 20, das die äußere Kontur des Produktes 12 abbildet (z.B. zweiter Datensatz) implementiert/integriert. Anders ausgedrückt wird die von der Röntgeneinheit 13 verwertbare produktspezifische Information über das optische Bild der optischen Kamera 17, 20 gelegt. Das dadurch entstehende Übergabebild (z.B. dritter Datensatz) ist letztlich ein Datenpaket mit allen für eine nachfolgende Verarbeitung notwendigen und von der Röntgeneinheit 13 und jeder optischen Kamera 17, 20 gewonnenen Informationen, wobei das Übergabebild ein optisches Bild ist.

Besonders bevorzugt ist die Steuerungseinheit 19 mit dem Prozessor 28 zum Bestimmen von Schnittpfaden aus den von der optischen Kamera 17, 20 erfassten produktspezifischen Informationen und aus den von der Röntgeneinheit 13 erfassten produktspezifischen Informationen konfiguriert, wobei die Schnittpfade aus den von der Röntgeneinheit 13 erfassten produktspezifischen Informationen eines Produktes 12 direkt über das optische Bild der optischen Kamera 17, 20 zum selben Produkt 12 gelegt werden, derart, dass die Steuerungseinheit 19 ein optisches Bild einer optischen Kamera 17, 20, nämlich das Übergabebild, zur Weiterverarbeitung bereitstellt. Es können auch separate Datensätze zur Weiterverarbeitung bereitgestellt werden, die von einer Steuerungseinheit einer nachgeordneten Verarbeitungsstation verarbeitbar sind.

Rein beispielhaft sind zwei mögliche Ausführungsformen anhand der Figuren 1 und 2 näher erläutert. Bei der Vorrichtung 10 gemäß Figur 1 kann z.B. mittels der optischen Kamera 17 und der angeschlossenen Steuerungseinheit 18 die äußere Kontur des Produktes 12 kreiert bzw. abgebildet werden (z.B. zweiter Datensatz), während mittels der Röntgeneinheit 13 und der angeschlossenen Steuerungseinheit 16 Schnittpfade für die vom Produkt 12 zu entfernenden Bereiche kreiert bzw. abgebildet werden (z.B. erster Datensatz). Durch Überlagerung des ersten Datensatzes auf den zweiten Datensatz, und zwar ohne jeden Abgleich ("matching"), wird der dritte Datensatz als optisches Bild kreiert bzw. abgebildet. Die Überlagerung kann z.B. alleine aufgrund der ermittelten Zeitdifferenz zwischen der Erzeugung des Röntgenbildes und des optischen Bildes erfolgen, und zwar als Funktion der Transportgeschwindigkeit des Transportförderers 11.

Bei der Vorrichtung 10 gemäß Figur 2 kann z.B. mittels der optischen Kamera 17, beispielsweise einer Grauwertkamera, und der angeschlossenen Steuerungseinheit 19 die äußere Kontur des Produktes 12 kreiert bzw. abgebildet werden. Mittels der optischen Kamera 20, beispielsweise einer Hyperspektralkamera, und der angeschlossenen Steuerungseinheit 19 können Schnittpfade für Fehlerstellen, die weder von der einfachen optischen Kamera 17 noch von der Röntgeneinheit 13 zuverlässig und präzise erkannt werden, nämlich z.B. Blutflecken, Farbveränderungen, Schnitte und dergleichen, und die vom Produkt 12 zu entfernen sind, kreiert bzw. abgebildet werden. Aus diesen Datensätzen bildet die Steuerungseinheit 19 ein Übergabebild als dritten Datensatz. Das Übergabebild kann auf der Basis des optischen Bildes der Grauwertkamera und/oder auf der Basis des optischen Bildes der Hyperspektralkamera, die zusätzlich zu den Schnittpfaden u.a. auch die äußere Kontur des Produktes 12 kreieren bzw. abbilden kann, erstellt werden. Das Übergabebild kann dann, optional angereichert um den von der Röntgeneinheit zur Verfügung gestellten Datensatz, für einen potentiellen Abgleich mit optischen Bildern nachgeordneter optischer Kameras verwendbar sein. Der von der Röntgeneinheit zur Verfügung gestellte Datensatz kann aber auch separat und unabhängig von den von der oder jeder Kamera zur Verfügung gestellten Datensatz zur weiteren Verarbeitung zur Verfügung gestellt werden.

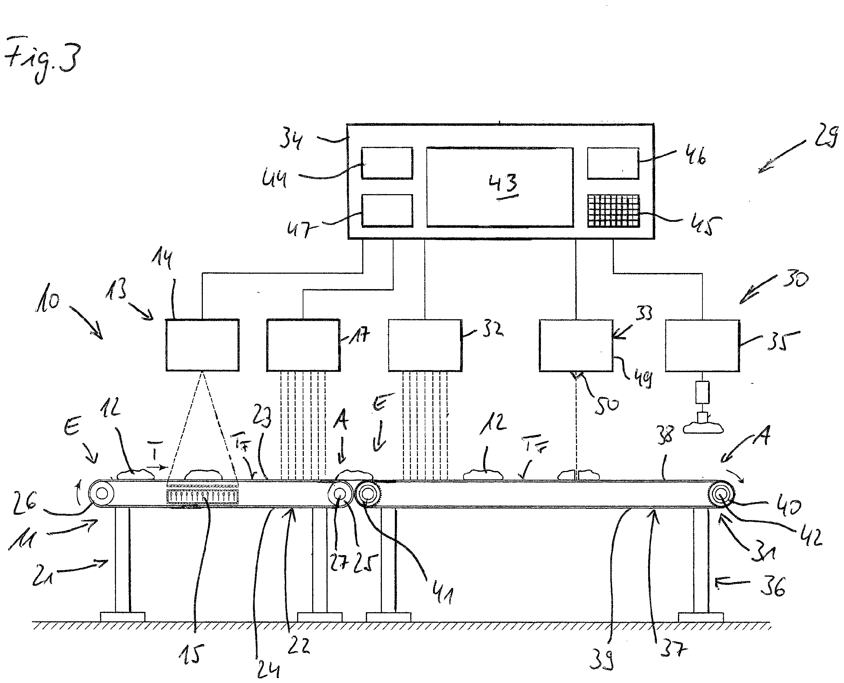

Wie bereits erwähnt, kann die Vorrichtung 10 als separate und mobile Vorrichtung zum Erfassen und Auswerten produktspezifischer Informationen eingesetzt werden. Bevorzugt ist die Vorrichtung 10 jedoch Bestandteil eines Systems 29 zum Verarbeiten von Produkten 12 der Nahrungsmittel verarbeitenden Industrie (siehe insbesondere Figur 3). Zur Verarbeitung von Produkten 12 zählt neben dem Sortieren und Verpacken insbesondere auch das Schneiden, wobei das Schneiden einerseits das Entfernen unerwünschter Bereiche von einem Produkt 12 und/oder andererseits das Teilen/Portionieren von Produkten 12 umfasst.

Das System 29 umfasst eine Vorrichtung 10, ausgebildet und eingerichtet zum Erfassen und Auswerten von produktspezifischen Informationen der Produkte 12, sowie eine in Transportrichtung T der Produkte 12 hinter der Vorrichtung 10 angeordnete Verarbeitungsstation 30, die einen Transportförderer 31 zum Transportieren der Produkte 12 in Transportrichtung T von einem Einlaufende E zu einem Auslaufende A, mindestens eine optische Kamera 32, mittels der produktspezifische Informationen von den auf dem Transportförderer 31 der Verarbeitungsstation 30 transportierten Produkten 12 erfassbar sind, sowie eine Trenneinheit 33 umfasst. Die optische Kamera 32 ist mit einer Steuerungseinheit 34 verbunden, die zum Empfangen und Auswerten der von der optischen Kamera 32 erfassten produktspezifischen Informationen ausgebildet und eingerichtet ist. Die Trenneinheit 33 ist zum Schneiden und Entfernen unerwünschter Bereiche der Produkte 12 und/oder zum Portionieren der Produkte 12 ausgebildet und eingerichtet, wobei die Trenneinheit 33 mit einer Steuerungseinheit 34 zum Steuern der Trenneinheit 33 auf der Basis zuvor erfasster und ausgewerteter produktspezifischer Informationen verbunden ist.

Dieses System 29 zeichnet sich erfindungsgemäß dadurch aus, dass die Vorrichtung 10, ausgebildet und eingerichtet zum Erfassen und Auswerten von produktspezifischen Informationen der Produkte 12, in einer Weise bzw. einer der Ausführungsformen ausgebildet ist, wie sie zuvor beschrieben wurden.

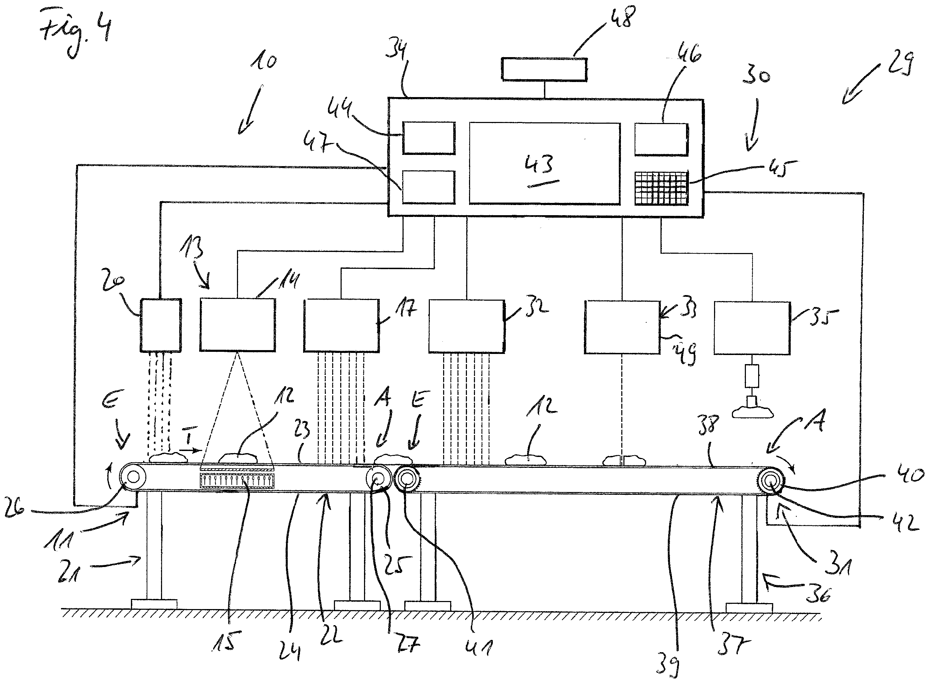

Eine erste Ausführungsform des Systems 29 ist in Figur 3 dargestellt. Die Vorrichtung 10 umfasst neben dem Transportförderer 11 eine Röntgeneinheit 13 und eine optische Kamera 17. Die Röntgeneinheit 13 und die optische Kamera 17 sind im Ausführungsbeispiel an die Steuerungseinheit 34 angeschlossen. Optional kann auch der Transportförderer 11 an die Steuerungseinheit 34 angeschlossen sein (siehe z.B. Figur 4). Alle Leitungs-/Signalverbindungen im System 29 können auf unterschiedliche Weise realisiert sein, nämlich z.B. draht-/leitungsgebunden oder leitungslos, z.B. über Funk- oder Bluetooth-Schnittstellen oder dergleichen. Die Verarbeitungsstation 30 umfasst neben dem Transportförderer 31 eine optische Kamera 32 und die Trenneinheit 33. Optional umfasst die Verarbeitungsstation 30 auch noch eine Entnahmestation 35 zum Entnehmen der Produkte 12 oder Teilen davon vom Transportförderer 31. Optische Kamera 32, Trenneinheit 33 und Entnahmestation 35 sind im Ausführungsbeispiel an die Steuerungseinheit 34 angeschlossen. Optional kann auch der Transportförderer 31 an die Steuerungseinheit 34 angeschlossen sein (siehe z.B. Figur 4). Alle Komponenten können auch an separate Steuerungseinheiten angeschlossen sein, die mit einer übergeordneten Steuerungseinheit in Wirkverbindung stehen. Auch besteht die Möglichkeit, dass einzelne oder sämtliche Komponenten der Vorrichtung 10 mit einer Steuerungseinheit verknüpft sind, die ihrerseits mit einer Steuerungseinheit der Verarbeitungsstation 30 in Wirkverbindung steht.

Der Transportförderer 31 der Verarbeitungsstation 30 weist ein Gestell 36 zum Stützen eines Transportförderbandes auf. Das Transportförderband ist bevorzugt ein umlaufend angetriebenes Förderband 37, das eine offene bzw. gitterartige Struktur aufweist. Vorzugsweise ist das Förderband 37 aus Edelstahl oder einem anderen robusten, rostfreien Material hergestellt. Das Förderband 37 weist einen Transportbandabschnitt 38 als Obertrum und einen Rückführbandabschnitt 39 als Untertrum auf. Das Förderband 37 mit seiner nach oben weisenden Transportfläche T_{F} umfasst Öffnungen, Durchbrüche oder dergleichen. Anders ausgedrückt ist die Transportfläche T_{F} bevorzugt über die gesamte Länge und Breite insbesondere für Wasser durchlässig ausgebildet. Das vorzugsweise endlos ausgebildete Förderband 37 ist um mindestens zwei Umlenkelemente 40, 41 gelenkt, von denen ein Umlenkelement 40 oder 41 als Antriebsrolle und das andere Umlenkelement 41 oder 40 als Umlenkrolle ausgebildet ist. Insbesondere im Bereich der Antriebsrolle, die mittels eines Antriebsmittels antreibbar ist, kann optional ein Encoder 42 vorgesehen sein, mittels dem die Position des Förderbandes 37 entlang der Länge des Transportförderers 31 und damit die Position des Produktes 12 auf dem Transportförderer 31 bestimmt bzw. überwacht werden kann.

Das System 29 gemäß Figur 4 ist vergleichbar mit dem System 29 gemäß Figur 3, weswegen für gleiche Komponenten die gleichen Bezugszahlen verwendet werden. Zusätzlich zum System 29 gemäß Figur 3 umfasst das System 29 gemäß Figur 4 für die Vorrichtung 10 eine zweite optische Kamera 20, die ebenfalls an die Steuerungseinheit 34 angeschlossen ist. Des Weiteren sind die Transportförderer 11, 31 - wie bereits weiter oben erwähnt - an die Steuerungseinheit 34 angeschlossen.

Bevorzugt stehen für eine oder jede Ausführungsform sämtliche Steuerungseinheiten des Systems 29 miteinander in Wirkverbindung bzw. bilden eine gemeinsame Steuerungseinheit 34. Diese Steuerungseinheit 34 umfasst mindestens einen Prozessor 43, der konfiguriert ist, optische Bilder der optischen Kameras 17, 20, 32 der Vorrichtung 10 einerseits und der Verarbeitungsstation 30 andererseits übereinanderzulegen, derart, dass das Identifizieren der Produkte 12 sowie das Abgleichen der relativen Positionen jedes Produktes 12 auf den beiden Transportförderern 11, 31 der Vorrichtung 10 einerseits und der Verarbeitungsstation 30 andererseits ausschließlich anhand optischer Bilder optischer Kameras 17, 20, 32 erfolgt. Die Steuerungseinheit 34 umfasst neben dem mindestens einen Prozessor 43 optional einen Speicher 44, eine Eingabeeinrichtung 45, eine Ausgabeeinrichtung 46 sowie eine Schnittstelle 47. Des Weiteren besteht die Möglichkeit, dass das System 29 über die Steuerungseinheit 34 mit einem Netzwerk 48, das intern (Intranet) oder extern (Internet) vernetzt sein kann, verbunden ist. Mittels der Steuerungseinheit 34 und dem Prozessor 43 können demnach die Bilddaten der optischen Bilder der Vorrichtung 10 einerseits und der Verarbeitungsstation 30 andererseits zur Identifizierung verglichen und zum Ermitteln der relativen Positionen jedes Produktes 12 auf den beiden Transportförderern 11, 31 abgeglichen und ausgewertet werden. Mittels der Steuerungseinheit 34 ist es aufgrund der Konfiguration des Prozessors 43 des Weiteren möglich, Transformationen zwischen den Bilddaten auszuführen.

In einer bevorzugten Ausführungsform sind die Vorrichtung 10 und die Verarbeitungsstation 30 an eine Steuerungseinheit 34 angeschlossen, die einen Prozessor 43 umfasst, der konfiguriert ist, ein optisches Übergabebild, das aus mindestens einem optischen Bild einer optischen Kamera 17 und/oder 20 der Vorrichtung 10, angereichert um von der Röntgeneinheit 13 der Vorrichtung 10 erfasste produktspezifische Daten, gebildet ist, und ein optisches Bild der optischen Kamera 32 der Verarbeitungsstation 30 übereinanderzulegen, derart, dass die Trenneinheit 33 mittels der Steuerungseinheit 34 für jedes Produkt 12 auf der Basis individuell ermittelter Schnittpfade steuerbar ist.

In einer bevorzugten Ausführungsform umfasst die Trenneinheit 33 eine Wasserstrahleinheit 49 mit mindestens einer Düse 50. Die oder jede Düse 50 ist optional frei im Raum steuerbar, um beliebigen Schnittpfaden folgen zu können. Anstelle der Wasserstrahleinheit 49 können auch andere Trennmittel, wie z.B. Trennmesser, Klingen, Bandmesser und dergleichen ansteuerbar sein.

Vorzugsweise ist mindestens je eine optische Kamera 17, 20, 32 in der Vorrichtung 10 einerseits und der Verarbeitungsstation 30 andererseits mit dem gleichen Bildgebungsverfahren ausgestattet. Besonders bevorzugt ist eine Ausführungsform, bei der die Vorrichtung 10 eine einfache optische Kamera 17, z.B. eine Grauwertkamera zur Aufnahme der äußeren Kontur, und eine komplexe optische Kamera 20, z.B. einer Hyperspektralkamera zur Aufnahme von Fehlerstellen, aufweist, und die Verarbeitungsstation 30 eine einfache optische Kamera 32, z.B. auch eine Grauwertkamera aufweist. Der Abgleich ("mapping") kann dann auf besonders einfache Weise zwischen zwei gleichen Bildgebungssystemen (Grauwertverfahren) erfolgen. In diesem Fall bildet das optische Bild der optischen Kamera 17 der Vorrichtung 10 die Basis für das Übergabebild (dritter Datensatz), in das die Informationen (Schnittpfade) der Röntgeneinheit 13 und der zweiten optischen Kamera 20 der Vorrichtung 10 eingearbeitet werden. Der Abgleich kann auch alleine auf der Basis z.B. des optischen Bildes der optischen Kamera 17 erfolgen, während die Datensätze der Röntgeneinheit 13 und der anderen optischen Kamera 20 zur Darstellung der Schnittpfade direkt auf das optischen Bild der Kamera 32 der Verarbeitungsstation 30 überlagert werden.

In einer weiteren nicht dargestellten Ausführungsform kann im Übergang von Transportförderer 11 der Vorrichtung 10 zu Transportförderer 31 der Verarbeitungsstation 30 eine zusätzliche optische Kamera angeordnet sein. Die optische Kamera nimmt das Auslaufende des Transportförderers 11 der Vorrichtung 10 und das Einlaufende des Transportförderers 31 der Verarbeitungsstation 30 dazu gleichzeitig auf. Diese optische Kamera ist vorzugsweise ebenfalls an die Steuerungseinheit 34 angeschlossen. Mit einer solchen optischen Kamera besteht die Möglichkeit, auf einen Abgleich ("matching") zwischen zwei Bilddaten (also Bilddaten der optischen Kamera 32 der Verarbeitungsstation 30 und der optischen Kamera 17 und/oder 20 der Vorrichtung 10) zu verzichten und eine Transformationsvorschrift zu berechnen, indem die Produktbewegung während der Übergabe von einem Transportförderer 11 auf den nachfolgenden Transportförderer 31 mittels der optischen Kamera beobachtet wird.

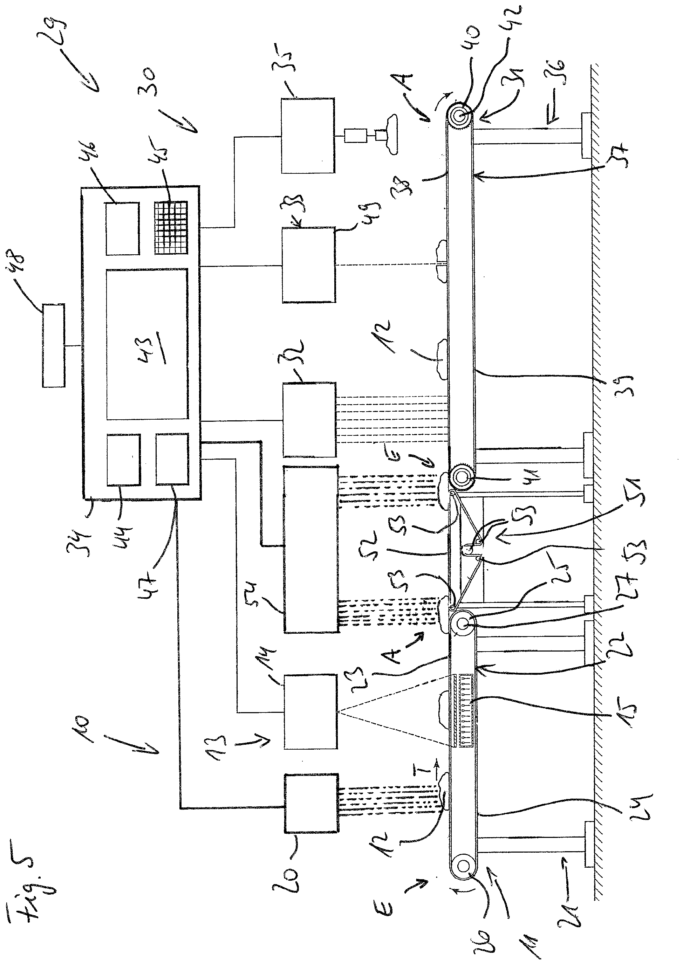

In der Figur 5 ist eine weitere Ausführungsform des Systems 29 gezeigt, die grundsätzlich ähnlich zu den Ausführungsformen der Figuren 3 und 4 ausgebildet ist. Allerdings weist das System 29 der Ausführungsform gemäß Figur 5 zwischen dem Transportförderer 11 der Vorrichtung 10 und dem Transportförderer 31 der Verarbeitungsstation 30 einen Zwischenförderer 51 auf, um die Übergabe der Produkte 12 von der Vorrichtung 10 an die Verarbeitungsstation 30 zu optimieren. Der Zwischenförderer 51 weist ein endloses Förderband 52 auf, das um mehr als zwei Umlenkelemente 53 umgelenkt ist. Mindestens eines der Umlenkelemente 53 ist als Antriebsrolle ausgebildet. Weitere Umlenkelemente 53 dienen dazu, die Spannung des Förderbandes 52 einstellen zu können. Die am Einlaufende E und Auslaufende A des Zwischenförderers 51 angeordneten Umlenkelemente 53 sind Umlenkrollen, deren äußere Durchmesser gegenüber den äußeren Durchmessern der Umlenkelemente 25, 41 der benachbarten Transportförderer 11, 31 relevant kleiner sind. Dadurch ist der Abstand zwischen dem Förderband 52 und dem Transportband 23 auf der einen Seite und dem Transportband 38 auf der anderen Seite reduziert, so dass der Transportförderer 11 der Vorrichtung 10, der Zwischenförderer 51 und der Transportförderer 31 der Verarbeitungsstation eine nahezu durchgängige und spaltfreie Transportfläche bilden. Sämtliche Transportförderer 11, 31 und Zwischenförderer 51 sind vorzugsweise mit derselben Geschwindigkeit angetrieben. Der Zwischenförderer 51 kann optional auch einen bestehenden Höhenversatz zwischen dem Transportförderer 11 der Vorrichtung 10 und dem Transportförderer 31 der Verarbeitungsstation 30 ausgleichen.

Der Zwischenförderer 51 ist vorzugsweise ebenfalls mit der Steuerungseinheit 34 verbunden. In einer Weiterbildung dieser Ausführungsform kann dem Zwischenförderer 51 mindestens eine optische Kamera 54 zugeordnet sein. In der Figur 5 ist eine Option gezeigt, in der die optische Kamera 54 beide Übergänge vom Transportförderer 11 auf den Zwischenförderer 51 und vom Zwischenförderer 51 auf den Transportförderer 31 aufnimmt. Andere Anordnungen und Ausbildungen der optischen Kameras sind ebenfalls möglich.

Neben den ausführlich beschriebenen optischen Kameras des Systems 29 als Bildgebungssystem können selbstverständlich z.B. auch 3D-Systeme und andere Bildgebungssysteme eingesetzt werden.

Im Folgenden wird das Verfahrensprinzip anhand der Zeichnung näher erläutert: Das Verfahren dient zum Verarbeiten von Produkten 12 der Nahrungsmittel verarbeitenden Industrie. Es ist für das Schneiden von Fischfilets beschrieben. Das Verfahren ist jedoch in gleicher Weise zum Schneiden anderer Produkte 12, wie z.B. Hühnchenfilets oder dergleichen, sowie zum Sortieren oder Verpacken nach produktspezifischen Informationen einsetzbar.

Zunächst werden die Produkte 12 mittels eines ersten Transportförderers 11 von einem Einlaufende E zu einem Auslaufende A in Transportrichtung T transportiert. Mittels einer Röntgeneinheit 13 werden zu jedem Produkt 12 auf dem ersten Transportförderer 11 produktspezifische Informationen, insbesondere Fehlerstellen in Form von Gräten, Grätenbereichen und anderes Hartgewebe erfasst, wobei die erfassten Informationen, die einen ersten Datensatz bilden, von einer Steuerungseinheit 34 empfangen und von dieser ausgewertet werden, um Schnittpfade zum Herausschneiden unerwünschter Bereiche aus den Produkten 12, z.B. aus Fischfilets, und/oder zum Portionieren der Produkte 12 zu bestimmen. Die Produkte 12 werden dann vom ersten Transportförderer 11 an einen nachfolgenden Transportförderer 31 weitergegeben, mittels dem die Produkte 12 in Transportrichtung T von einem Einlaufende zu einem Auslaufende transportiert werden. Auf dem nachfolgenden Transportförderer 31 werden mittels einer optischen Kamera 32 produktspezifische Informationen erfasst, wobei die erfassten Informationen, insbesondere die äußere Kontur, die einen vierten Datensatz bilden, von einer Steuerungseinheit 34 empfangen und von dieser ausgewertet werden, um jedes Produkt zu identifizieren. Erfindungsgemäß werden auf dem ersten Transportförderer 11 zusätzlich zu den von der Röntgeneinheit 13 erfassten produktspezifischen Informationen produktspezifische Informationen zu jedem Produkt 12 mittels mindestens einer optischen Kamera 17 und/oder 20 erfasst, wobei die durch die dem ersten Transportförderer 11 zugeordnete optische Kamera 17 und/oder 20 erfassten Informationen, die einen zweiten Datensatz bilden, von einer Steuerungseinheit 34 empfangen und ausgewertet werden. Des Weiteren werden die empfangenen Datensätze mittels einer Steuerungseinheit 34 abgeglichen und ausgewertet, um die relativen Produktpositionen jedes Produktes 12 auf den beiden Transportförderern 11, 31 zu berechnen. Schließlich werden die Produkte 12 entlang der zuvor ermittelten und dem jeweiligen Produkt 12 zugeordneten Schnittpfade mittels einer Trenneinheit 33 geschnitten, wobei die Trenneinheit 33 durch eine Steuerungseinheit 34 gesteuert wird. Die empfangenen Datensätze werden weiterhin dazu verwendet, mögliche Transformationen auszuführen, die aufgrund von Verschiebungen, Bewegungen, Verwindungen oder dergleichen der Produkte 12 bei der Übergabe der Produkte 12 von einem Transportförderer 11 auf einen anderen Transportförderer 31 auftreten.

Wie erwähnt, existieren zum Identifizieren und Abgleichen und Auswerten der gesammelten Informationen mehrere Optionen. Das Identifizieren der Produkte und das Abgleichen kann z.B. alleine auf der Basis des Bildes einer der optischen Kameras 17, 20 der Vorrichtung 10 sowie des Bildes der optischen Kamera 32 der Verarbeitungsstation 30 erfolgen. Ist das Produkt 12 identifiziert und deren Lage/Position auf dem zweiten Transportförderer 31 ggf. nach einer Transformation bekannt, können die Schnittpfade, generiert aus den Datensätze der Röntgeneinheit 13 und einer der optischen Kameras 17, 20 der Vorrichtung 10, in das Bild der optischen Kamera 32 der Verarbeitungsstation 30 integriert/implementiert werden. Vor dem Vergleich/Abgleich des Bildes einer der optischen Kameras 17, 20 der Vorrichtung 10 mit dem Bild der optischen Kamera 32 der Verarbeitungsstation 30 können die Schnittpfade, generiert aus den Datensätze der Röntgeneinheit 13 und einer der optischen Kameras 17, 20 der Vorrichtung 10, in das Bild der weiteren optischen Kamera 17, 20 der Vorrichtung 10 integriert/implementiert werden.

Das Identifizieren der Produkte 12 und das Abgleichen und das Auswerten der Datensätze erfolgt auf der Basis der von den optischen Kameras 17 und/oder 20 und 32 erzeugten Datensätzen, nämlich vorzugsweise dem zweiten Datensatz und dem vierten Datensatz. Die empfangenen Datensätze werden entsprechend auch dazu verwendet, die Positionen der zu entfernenden Bereiche aus dem Produkt 12 und/oder die Schnittlinien zum Portionieren der Produkte 12 für das jeweilige auf dem Transportförderer 31 der Verarbeitungsstation 30 liegende und identifizierte Produkt 12 abzubilden, um die Trenneinheit 33 zu steuern.

Mit anderen Worten wird erfindungsgemäß für den Abgleich ("matching") ein optisches Bild von der Vorrichtung 10 an die Steuerungseinheit 34 übertragen und dieses optische Bild der Vorrichtung 10 mit dem optischen Bild der Verarbeitungsstation 30 abgeglichen. Das bedeutet, dass nach dem Verarbeiten aller Bilddaten (Röntgenbild und optisches Bild), die von den Produkten 12 auf dem ersten Transportförderer 11 aufgenommen werden, diese für den Abgleich-Prozess ("matching") an die Steuerungseinheit 34 (CPU) übertragen werden. Vorzugsweise werden diese für jedes Produkt 12 individuellen Bilddaten in einer Liste gespeichert. Nachdem jedes Produkt 12 auf dem zweiten Transportförderer 31 mittels der optischen Kamera 32 erfasst wurde, wird das optische Bild der optischen Kamera 32 mit den Bilddaten aus der Liste vergleichen. Es wird/werden dasjenige Bild bzw. diejenigen Bilddaten aus der Liste ausgewählt, welches/welche die größten Übereinstimmungen aufweisen. Nach erfolgreichem Abgleich ("matching") des optischen Bildes der optischen Kamera 32 mit einem Bild/Bilddaten aus der Liste werden dieses Bild/diese Bilddaten aus der Liste entfernt. Mit anderen Worten wird zum Identifizieren der Produkte 12 jedes mit der dem nachfolgenden Transportförderer 31 zugeordneten optischen Kamera 32 aufgenommene optische Bild mit den in einem Speicher 44 (auch als Liste bezeichnet) einer Steuerungseinheit 34 gespeicherten optischen Bildern der dem ersten Transportförderer 11 zugeordneten optischen Kamera 17 und/oder 20 verglichen und das Bild mit den größten Übereinstimmungen ausgewählt. Nach dem erfolgreichen Identifizieren und ggf. Abgleichen und Auswerten wird das entsprechende optische Bild aus dem Speicher gelöscht.

Sollte kein (passendes) Bild/keine (passenden) Bilddaten in der Liste bzw. im Speicher 44 vorhanden sein, oder kein Bild/keine Bilddaten aus dem Speicher 44 ein ausreichendes Abgleich-Ergebnis erzielen, kann das Produkt 12 in der Verarbeitung übersprungen und beispielsweise zur manuellen Bearbeitung transportiert werden. Sollte ein Bild/Bilddaten länger in dem Speicher 44 verweilen als plausibel, kann es optional automatisch aus dem Speicher 44 entfernt werden.

Der erste Datensatz, der die durch die Röntgeneinheit 13 generierten Schnittpfade beinhaltet, wird in den zweiten Datensatz, nämlich in das durch die dem ersten Transportförderer 11 zugeordnete optische Kamera 17 oder 20 generierte optische Bild integriert/implementiert, so dass ein optisches Übergabebild als dritter Datensatz zum Abgleichen und Auswerten an die Steuerungseinheit 34 weitergegeben wird, wobei das Identifizieren der Produkte 12 und das Abgleichen und das Auswerten der Datensätze auf der Basis des dritten und des vierten Datensatzes erfolgt. Das bedeutet, dass die Bilddaten der optischen Kamera 17 oder 20 (zusammen mit den von der Röntgeneinheit 13 gewonnenen Informationen) an die Steuerungseinheit 34 übertragen werden. Die Steuerungseinheit 34 empfängt des Weiteren die Bilddaten der optischen Kamera 32 und verarbeitet sämtliche Bilddaten nach der Zuordnung zu einem Produkt 12 zur Steuerung der Trenneinheit 33. Optional können die Daten der Röntgeneinheit 13, die Bilddaten der optischen Kamera 17 oder 20 und die Bilddaten der optischen Kamera 32 separat an die Steuerungseinheit 34 übermittelt werden. Die Daten der Röntgeneinheit 13 können dann über die Bilddaten der optischen Kamera 32 der Verarbeitungsstation 30 überlagert werden. Anschließend kann ein Vergleich der Bilddaten der optischen Kamera 17 oder 20 der Vorrichtung 10 mit den Bilddaten der optischen Kamera 32 der Verarbeitungsstation 30 durchgeführt werden, um sicherzustellen, dass das Produkt 12 das identische ist. Dann kann, falls erforderlich, ein Transformieren der Daten der Röntgeneinheit 13 und/oder der optischen Kamera 17, 20 der Vorrichtung 10 z.B. durch X-Y-Translation, Rotation, X-Y- Scherung, X-Y-Verschiebung oder dergleichen vom optischen Bild der Kamera 17 oder 20 der Vorrichtung 10 zum optischen Bild der Kamera 32 der Verarbeitungsstation 30 erfolgen. Die Reihenfolge dieser Schritte kann variieren.

Der Abgleich ("matching") selbst kann beispielhaft wie folgend erläutert, ausgeführt werden: Es werden markante Punkte in den optischen Bildern der optischen Kameras 17 oder 20 und 32 gesucht und anhand ihrer relativen Lage einander so zugeordnet, dass relative Lagen/Nachbarschaften möglichst gut abgebildet werden. Dabei wird eine Transformations-Funktion bestimmt, welche ein rechteckiges Gitter über dem optischen Bild der optischen Kamera 17 oder 20 der Vorrichtung 10 in ein splineförmiges Gitter über dem optischen Bild der optischen Kamera 32 der Verarbeitungsstation 30 abbildet. Mittels der Transformationsfunktion wird jedem Punkt im Koordinatensystem des optischen Bildes der optischen Kamera 17 oder 20 der Vorrichtung 10 ein eindeutiger Punkt im Koordinatensystem des optischen Bildes der optischen Kamera 32 der Verarbeitungsstation 30 zugeordnet. Das Abgleich-Ergebnis wird über die Ähnlichkeit der gesamten Bilddaten des Produktes 12 in den Bilddaten der optischen Kameras 17 oder 20 und 32 bestimmt, wobei die Transformations-Funktion dazu verwendet wird, um die Punkte einander zuzuordnen. Ähnlichkeit ist dabei im Sinne von möglichst geringen Abweichungen bezüglich des entsprechenden Bildgebungsverfahrens zu verstehen.

Vorzugsweise werden die Positionen der durch die Röntgeneinheit 13 und/oder eine weitere optische Kamera 17, 20 generierten, unerwünschten Bereiche als Vektordaten, Pixeldaten oder dergleichen in das optische Bild der dem ersten Transportförderer 11 zugeordneten optischen Kamera 17, 20 integriert/implementiert, wobei das Abgleichen ("matching") und Auswerten aufgrund des optischen Bildes der dem ersten Transportförderer 11 zugeordneten optischen Kamera 17, 20 und des optischen Bildes der dem zweiten Transportförderer 31 zugeordneten optischen Kamera 32 ausgeführt wird. Es besteht also die Möglichkeit, dass das optische Bild zusätzlich zum Identifizieren (z.B. über die äußere Kontur des Produktes) auch zum Generieren von Schnittpfaden verwendet wird.

Besonders bevorzugt wird das beschriebene Verfahren mit einem System 29, wie es zuvor beschrieben wurde, ausgeführt.

Der Schutzumfang der vorliegenden Erfindung wird durch die angefügten Ansprüche definiert.

## Patentansprüche

1. Vorrichtung (10), ausgebildet und eingerichtet zum Erfassen und Auswerten von produktspezifischen Informationen von Produkten (12) der Nahrungsmittel verarbeitenden Industrie, umfassend einen in der Transportebene spaltfreien Transportförderer (11) zum Transportieren vereinzelter Produkte (12) in Transportrichtung T von einem Einlaufende zu einem Auslaufende, eine Röntgeneinheit (13) mit mindestens einer Röntgenquelle (14) und mindestens einer Röntgenkamera (15) zum Erfassen produktspezifischer Informationen, wobei Röntgenquelle (14) und Röntgenkamera (15) dem Transportförderer (11) derart zugeordnet sind, dass die Produkte (12) zwischen der Röntgenquelle (14) und der Röntgenkamera (15) entlang führbar sind, sowie eine Steuerungseinheit (16, 19), die mit der Röntgeneinheit (13) verbunden und zum Empfangen und Auswerten der von der Röntgeneinheit (13) erfassten, produktspezifischen Informationen, die einen ersten Datensatz bilden, ausgebildet und eingerichtet ist, wobei demselben Transportförderer (11) zwischen dessen Einlaufende und Auslaufende mindestens eine optische Kamera (17, 20) zugeordnet ist, mittels der zusätzlich zu der Röntgeneinheit (13) produktspezifische Informationen von den auf dem Transportförderer (11) transportierten Produkten (12) erfassbar sind, wobei die optische Kamera (17) mit einer Steuerungseinheit (18, 19) verbunden ist, die zum Empfangen und Auswerten der von der optischen Kamera (17, 20) erfassten, produktspezifischen Informationen, die einen zweiten Datensatz bilden, ausgebildet und eingerichtet ist, **dadurch gekennzeichnet, dass** die Röntgeneinheit (13) und jede optische Kamera (17, 20) sowie der Transportförderer (11) an eine Steuerungseinheit (19) zum Empfangen und Auswerten der Datensätze angeschlossen sind, wobei die Steuerungseinheit (19) mit mindestens einem Prozessor (28) ausgestattet ist, der zum Integrieren/Implementieren mindestens von Teilen der durch die Röntgeneinheit (13) erfassten produktspezifischen Informationen eines Produktes (12) in das optische Bild der optischen Kamera (17, 20) zum selben Produkt (12) zur Bildung eines optischen Übergabebildes, das einen dritten Datensatz bildet, konfiguriert ist.

2. Vorrichtung (10) nach Anspruch 1, **dadurch gekennzeichnet, dass** mindestens eine optische Kamera (17, 20) oberhalb des Transportförderers (11) angeordnet ist.

3. Vorrichtung (10) nach Anspruch 1 oder 2, **dadurch gekennzeichnet, dass** dem Transportförderer (11) mindestens zwei optische Kameras (17, 20) zugeordnet sind, die mit einem unterschiedlichen Bildgebungsverfahren ausgestattet sind.

4. Vorrichtung (10) nach einem oder mehreren der Ansprüche 1 bis 3, **dadurch gekennzeichnet, dass** mindestens eine optische Kamera (17, 20) eine Multi- oder Hyperspektralkamera ist.

5. Vorrichtung (10) nach einem oder mehreren der Ansprüche 1 bis 4, **dadurch gekennzeichnet, dass** mindestens eine optische Kamera (17, 20) als Grauwertkamera und/oder RGB-Kamera und/oder IR- und/oder UV-Kamera ausgebildet ist.

6. Vorrichtung (10) nach einem oder mehreren der Ansprüche 1 bis 5, **dadurch gekennzeichnet, dass** der Transportförderer (11) ein umlaufend angetriebenes und auf der Transportfläche T_{F} strukturarmes Röntgenförderband (22), das aus Kunststoff hergestellt ist, mit einem Transportband (23) als Obertrum und einem Rückführband (24) als Untertrum ist.

7. Vorrichtung (10) nach einem oder mehreren der Ansprüche 1 bis 6, **dadurch gekennzeichnet, dass** der Transportförderer (11) an eine Steuerungseinheit (19) angeschlossen ist, die zum Empfangen und Auswerten von Bewegungsdaten des Transportförderers (11) ausgebildet und eingerichtet ist.

8. Vorrichtung (10) nach Anspruch 7, **dadurch gekennzeichnet, dass** die Röntgenquelle (14) oberhalb des Transportbandes (23) und die Röntgenkamera (15) zwischen dem Transportband (23) und dem Rückführband (24) angeordnet ist.

9. Vorrichtung (10) nach einem oder mehreren der Ansprüche 1 bis 8, **dadurch gekennzeichnet, dass** die Steuerungseinheit (19) mit dem Prozessor (28) zum Bestimmen von Schnittpfaden aus den von der optischen Kamera (17, 20) erfassten produktspezifischen Informationen und aus den von der Röntgeneinheit (13) erfassten produktspezifischen Informationen konfiguriert ist, wobei die Schnittpfade aus den von der Röntgeneinheit (13) erfassten produktspezifischen Informationen eines Produktes (12) direkt über das optische Bild der optischen Kamera (17, 20) zum selben Produkt (12) gelegt werden, derart, dass die Steuerungseinheit (19) ein optisches Bild einer optischen Kamera (17, 20), nämlich das Übergabebild, zur Weiterverarbeitung bereitstellt.

10. System (29), ausgebildet und eingerichtet zum Verarbeiten von Produkten (12) der Nahrungsmittel verarbeitenden Industrie, umfassend eine Vorrichtung (10), ausgebildet und eingerichtet zum Erfassen und Auswerten von produktspezifischen Informationen der Produkte (12), sowie eine in Transportrichtung T der Produkte (12) hinter der Vorrichtung (10) angeordnete Verarbeitungsstation (30), die einen Transportförderer (31) zum Transportieren der Produkte (12) in Transportrichtung T von einem Einlaufende zu einem Auslaufende, mindestens eine optische Kamera (32), mittels der produktspezifische Informationen von den auf dem Transportförderer (31) der Verarbeitungsstation (30) transportierten Produkten (12) erfassbar sind, wobei die optische Kamera (32) mit einer Steuerungseinheit (34) verbunden ist, die zum Empfangen und Auswerten der von der optischen Kamera (34) erfassten produktspezifischen Informationen ausgebildet und eingerichtet ist, sowie eine Trenneinheit (33) umfasst, die zum Schneiden und Entfernen unerwünschter Bereiche der Produkte (12) und/oder zum Portionieren der Produkte (12) ausgebildet und eingerichtet ist, wobei die Trenneinheit (33) mit einer Steuerungseinheit (34) zum Steuern der Trenneinheit (33) auf der Basis zuvor erfasster und ausgewerteter produktspezifischer Informationen verbunden ist, **dadurch gekennzeichnet, dass** die Vorrichtung (10), ausgebildet und eingerichtet zum Erfassen und Auswerten von produktspezifischen Informationen der Produkte (12), nach einem der Ansprüche 1 bis 9 ausgebildet ist.

11. System (29) nach Anspruch 10, **dadurch gekennzeichnet, dass** sämtliche Steuerungseinheiten (34) des Systems (29) miteinander in Wirkverbindung stehen und mindestens eine Steuerungseinheit (34) einen Prozessor (43) umfasst, der konfiguriert ist, optische Bilder der optischen Kameras (17, 20, 32) der Vorrichtung (10) einerseits und der Verarbeitungsstation (30) andererseits übereinanderzulegen, derart, dass das Identifizieren der Produkte (12) sowie das Abgleichen der relativen Positionen jedes Produktes (12) auf den beiden Transportförderern (11, 31) der Vorrichtung (10) einerseits und der Verarbeitungsstation (30) andererseits ausschließlich anhand optischer Bilder optischer Kameras (17, 20, 32) erfolgt.

12. System (29) nach Anspruch 10 oder 11, **dadurch gekennzeichnet, dass** die Vorrichtung (10) und die Verarbeitungsstation (30) an eine Steuerungseinheit (34) angeschlossen sind, die einen Prozessor (43) umfasst, der konfiguriert ist, ein optisches Übergabebild, das aus einem optischen Bild einer optischen Kamera (17, 20) der Vorrichtung (10), angereichert um von der Röntgeneinheit (13) der Vorrichtung (10) erfasste produktspezifische Daten, gebildet ist, und ein optisches Bild der optischen Kamera (32) der Verarbeitungsstation (30) übereinanderzulegen, derart, dass die Trenneinheit (33) mittels der Steuerungseinheit (34) für jedes Produkt (12) auf der Basis individuell ermittelter Schnittpfade steuerbar ist.

13. System (29) nach einem oder mehreren der Ansprüche 10 bis 12, **dadurch gekennzeichnet, dass** die Trenneinheit (33) eine Wasserstrahleinheit (49) mit mindestens einer Düse (50) umfasst.

14. System (29) nach einem oder mehreren der Ansprüche 10 bis 13, **dadurch gekennzeichnet, dass** mindestens je eine optische Kamera (17, 32) in der Vorrichtung (10) einerseits und der Verarbeitungsstation (30) andererseits mit dem gleichen Bildgebungsverfahren ausgestattet ist.

15. System (29) nach einem oder mehreren der Ansprüche 10 bis 14, **dadurch gekennzeichnet, dass** mindestens eine Steuerungseinheit (34) mindestens einen Speicher (44), mindestens eine Eingabeeinrichtung (45) sowie mindestens eine Ausgabeeinrichtung (46) umfasst.

16. Verfahren zum Verarbeiten von Produkten (12) der Nahrungsmittel verarbeitenden Industrie, umfassend die Schritte:
- Transportieren der Produkte (12) mittels eines ersten Transportförderers (11) von einem Einlaufende zu einem Auslaufende in Transportrichtung T,
- Erfassen produktspezifischer Informationen zu jedem Produkt (12) auf dem ersten Transportförderer (11) mittels einer Röntgeneinheit (13), wobei die erfassten Informationen, die einen ersten Datensatz bilden, von einer Steuerungseinheit (16, 19) empfangen und von dieser ausgewertet werden, um Schnittpfade zum Herausschneiden unerwünschter Bereiche und/oder zum Portionieren zu bestimmen,
- Weitergeben der Produkte vom ersten Transportförderer (11) an einen nachfolgenden Transportförderer (31), mittels dem die Produkte (12) in Transportrichtung T von einem Einlaufende zu einem Auslaufende transportiert werden,
- Erfassen produktspezifischer Informationen zu jedem Produkt (12) auf dem nachfolgenden Transportförderer (31) mittels einer optischen Kamera (32), wobei die erfassten Informationen, die einen vierten Datensatz bilden, von einer Steuerungseinheit (34) empfangen und von dieser ausgewertet werden, um jedes Produkt (12) zu identifizieren,
- Abgleichen und Auswerten der empfangenen und ausgewerteten Datensätze mittels einer Steuerungseinheit (34), um die relativen Produktpositionen jedes Produktes (12) auf den beiden Transportförderern (11, 31) zu berechnen, und
- Schneiden der Produkte (12) entlang der zuvor ermittelten und dem jeweiligen Produkt zugeordneten Schnittpfade mittels einer Trenneinheit (33), wobei die Trenneinheit (33) durch eine Steuerungseinheit (34) gesteuert wird,
wobei auf dem ersten Transportförderer (11) zusätzlich zu den von der Röntgeneinheit (13) erfassten produktspezifischen Informationen produktspezifische Informationen zu jedem Produkt (12) mittels einer optischen Kamera (17, 20) erfasst werden, wobei die durch die dem ersten Transportförderer (11) zugeordnete optische Kamera (17, 20) erfassten Informationen, die einen zweiten Datensatz bilden, von einer Steuerungseinheit (18, 19, 34) empfangen und ausgewertet werden, **dadurch gekennzeichnet, dass** der erste Datensatz, der die durch die Röntgeneinheit (13) generierten Schnittpfade beinhaltet, in den zweiten Datensatz, nämlich in das durch die dem ersten Transportförderer (11) zugeordnete optische Kamera (17, 20) generierte optische Bild integriert/implementiert wird, so dass ein optisches Übergabebild als dritter Datensatz zum Abgleichen und Auswerten an die Steuerungseinheit (19, 34) weitergegeben wird, und dass das Identifizieren der Produkte (12) und das Abgleichen und das Auswerten der Datensätze auf der Basis des dritten und des vierten Datensatzes erfolgt.

17. Verfahren nach Anspruch 16, **dadurch gekennzeichnet, dass** die Positionen der durch die Röntgeneinheit (13) und/oder eine weitere optische Kamera (17, 20) generierten, unerwünschten Bereiche als Vektordaten, Pixeldaten oder dergleichen in das optische Bild der dem ersten Transportförderer (11) zugeordneten optischen Kamera (17, 20) integriert/implementiert werden, und dass das Abgleichen ("matching") und Auswerten aufgrund des optischen Bildes der dem ersten Transportförderer (11) zugeordneten optischen Kamera (17, 20) und des optischen Bildes der dem zweiten Transportförderer (31) zugeordneten optischen Kamera (32) ausgeführt wird.

18. Verfahren nach Anspruch 16 oder 17, **dadurch gekennzeichnet, dass** zum Identifizieren der Produkte jedes mit der dem nachfolgenden Transportförderer (31) zugeordneten optischen Kamera (32) aufgenommene optische Bild mit den in einem Speicher (44) einer Steuerungseinheit (34) gespeicherten optischen Bildern der dem ersten Transportförderer (11) zugeordneten optischen Kamera (17, 20) verglichen und das Bild mit den größten Übereinstimmungen ausgewählt wird.

19. Verfahren nach einem oder mehreren der Ansprüche 16 bis 18, **dadurch gekennzeichnet, dass** es mit einem System (29) nach einem der Ansprüche 10 bis 15 ausgeführt wird.

## Claims

1. Apparatus (10), designed and set up for detecting and evaluating product-specific information of products (12) of the food-processing industry, comprising a transport conveyor (11), free of gaps in the transport plane, for transporting individual products (12) in the transport direction T from an infeed end to an outfeed end, an X-ray unit (13) with at least one X-ray source (14) and at least one X-ray camera (15) for detecting product-specific information, the X-ray source (14) and X-ray camera (15) being assigned to the transport conveyor (11) in such a way that the products (12) can be guided along between the X-ray source (14) and the X-ray camera (15), and a control unit (16, 19) which is connected to the X-ray unit (13) and is designed and set up for receiving and evaluating the product-specific information which is recorded by the X-ray unit (13) and forms a first data set, wherein at least one optical camera (17, 20), by means of which, in addition to the X-ray unit (13), product-specific information can be detected from the products (12) transported on the transport conveyor (11), the optical camera (17) being connected to a control unit (18, 19), which is designed and set up to receive and evaluate the product-specific information detected by the optical camera (17, 20), which forms a second data set, **characterized in that** the X-ray unit (13) and each optical camera (17, 20) and the transport conveyor (11) are connected to a control unit (19) for receiving and evaluating the data records, the control unit (19) being equipped with at least one processor (28) which is configured for integrating/implementing at least parts of the product-specific information of a product (12) captured by the X-ray unit (13) into the optical image of the optical camera (17, 20) for the same product (12) to form an optical transfer image which forms a third data record.

2. Apparatus (10) according to claim 1, **characterized in that** at least one optical camera (17, 20) is arranged above the transport conveyor (11).

3. Apparatus (10) according to claim 1 or 2, **characterized in that** at least two optical cameras (17, 20), which are equipped with a different imaging method, are assigned to the transport conveyor (11).

4. Apparatus (10) according to one or more of claims 1 to 3, **characterized in that** at least one optical camera (17, 20) is a multi-spectral or hyperspectral camera.

5. Apparatus (10) according to one or more of claims 1 to 4, **characterized in that** at least one optical camera (17, 20) is designed as a grey-scale camera and/or RGB camera and/or IR camera and/or UV camera.

6. Apparatus (10) according to one or more of the claims 1 to 5, **characterized in that** the transport conveyor (11) is a circumferentially driven X-ray conveyor belt (22) which is made of plastic and has a low structure on the transport surface T_{F} , with a transport belt (23) as an upper run and a return belt (24) as an lower run.

7. Apparatus (10) according to one or more of claims 1 to 6, **characterized in that** the transport conveyor (11) is connected to a control unit (19) which is designed and set up for receiving and evaluating movement data of the transport conveyor (11).

8. Apparatus (10) according to claim 7, **characterized in that** the X-ray source (14) is arranged above the conveyor belt (23) and the X-ray camera (15) is arranged between the conveyor belt (23) and the return belt (24).

9. Apparatus (10) according to one or more of claims 1 to 8, **characterized in that** the control unit (19) is configured with the processor (28) for determining cut paths from the product-specific information captured by the optical camera (17, 20) and from the product-specific information captured by the X-ray unit (13), wherein the cut paths from the product-specific information of a product (12) recorded by the X-ray unit (13) are superimposed over the optical image of the optical camera (17, 20) for the same product (12) in such a way that the control unit (19) provides an optical image of an optical camera (17, 20), namely the transfer image, for further processing.

10. System (29), designed and set up for processing products (12) of the food processing industry, comprising a device (10), designed and set up for detecting and evaluating product-specific information of the products (12), and a processing station (30) arranged downstream of the apparatus (10) in the transport direction T of the products (12), which has a transport conveyor (31) for transporting the products (12) in the transport direction T from an infeed end to an outfeed end, at least one optical camera (32), by means of which product-specific information from the products (12) transported on the transport conveyor (31) of the processing station (30) can be detected, the optical camera (32) being connected to a control unit (34), which is designed and set up for receiving and evaluating the product-specific information recorded by the optical camera (34), and a separating unit (33) which is designed and set up for cutting and removing undesired areas of the products (12) and/or for portioning the products (12), the separating unit (33) being connected to a control unit (34) for controlling the separating unit (33) on the basis of previously detected and evaluated product-specific information, **characterized in that** the apparatus (10), designed and set up for detecting and evaluating product-specific information of the products (12), is designed according to one of claims 1 to 9.

11. System (29) according to claim 10, **characterized in that** all control units (34) of the system (29) are operatively connected to one another and at least one control unit (34) comprises a processor (43) which is configured to superimpose optical images of the optical cameras (17, 20, 32) of the apparatus (10) on the one hand and of the processing station (30) on the other hand, such that the identification of the products (12) and the comparison of the relative positions of each product (12) on the two transport conveyors (11, 31) of the apparatus (10) on the one hand and of the processing station (30) on the other hand is carried out exclusively on the basis of optical images from optical cameras (17, 20, 32).

12. System (29) according to claim 10 or 11, **characterized in that** the apparatus (10) and the processing station (30) are connected to a control unit (34) which comprises a processor (43) which is configured to superimpose an optical transfer image, which is formed from an optical image of an optical camera (17, 20) of the apparatus (10) enriched by product-specific data recorded by the X-ray unit (13) of the apparatus (10), and an optical image of the optical camera (32) of the processing station (30), in such a way that the separating unit (33) can be controlled by means of the control unit (34) for each product (12) on the basis of individually determined cutting paths.

13. System (29) according to one or more of claims 10 to 12, **characterized in that** the separating unit (33) comprises a water jet unit (49) with at least one nozzle (50).

14. System (29) according to one or more of claims 10 to 13, **characterized in that** at least one optical camera (17, 32) in each of the apparatus (10) on the one hand and the processing station (30) on the other hand is equipped with the same imaging method.

15. System (29) according to one or more of claims 10 to 14, **characterized in that** at least one control unit (34) comprises at least one memory (44), at least one input device (45) and at least one output device (46).

16. A method for processing products (12) of the food processing industry, comprising the steps of:
- transporting the products (12) by means of a first transport conveyor (11) from an infeed end to an outfeed end in transport direction T,
- detection of product-specific information for each product (12) on the first transport conveyor (11) by means of an X-ray unit (13), the detected information, which forms a first data set, being received by a control unit (16, 19) and evaluated by the latter in order to determine cutting paths for cutting out unwanted areas and/or for portioning,
- transfer of the products from the first transport conveyor (11) to a subsequent transport conveyor (31), by means of which the products (12) are transported in transport direction T from an infeed end to an outfeed end,
- capturing product-specific information about each product (12) on the downstream transport conveyor (31) by means of an optical camera (32), the captured information, which forms a fourth data set, being received by a control unit (34) and evaluated by the latter in order to identify each product (12),
- matching and evaluating the received and evaluated data records by means of a control unit (34) in order to calculate the relative product positions of each product (12) on the two transport conveyors (11, 31), and
- cutting of the products (12) along the cutting paths previously determined and assigned to the respective product by means of a separating unit (33), the separating unit (33) being controlled by a control unit (34),
wherein on the first transport conveyor (11), in addition to the product-specific information recorded by the X-ray unit (13), product-specific information on each product (12) is recorded by means of an optical camera (17, 20), wherein the information recorded by the optical camera (17, 20) assigned to the first transport conveyor (11), which contains the cutting paths generated by the X-ray unit (13), is integrated/implemented into the second data set, namely into the optical image generated by the optical camera (17, 20) assigned to the first transport conveyor (11), so that an optical transfer image is passed on to the control unit (19, 34) as a third data set for comparison and evaluation, and in that the identification of the products (12) and the comparison and evaluation of the data sets are carried out on the basis of the third and fourth data sets.

17. Method according to claim 16, **characterized in that** the positions of the unwanted areas generated by the X-ray unit (13) and/or a further optical camera (17, 20) are integrated/implemented as vector data, pixel data or the like into the optical image of the optical camera (17, 20) assigned to the first transport conveyor (11), and that the matching and evaluation is performed on the basis of the optical image of the optical camera (17, 20) assigned to the first transport conveyor (11) and the optical image of the optical camera (32) assigned to the second transport conveyor (31).

18. Method according to claim 16 or 17, **characterized in that**, in order to identify the products, each optical image recorded by the optical camera (32) assigned to the subsequent transport conveyor (31) is compared with the optical images stored in a memory (44) of a control unit (34) of the optical camera (17, 20) assigned to the first transport conveyor (11) and the image with the greatest matches is selected.

19. The method according to one or more of claims 16 to 18, **characterized in that** it is carried out with a system (29) according to one of claims 10 to 15.

## Revendications

1. Dispositif (10) configuré et adapté pour saisir et évaluer des informations spécifiques aux produits sur des produits (12) de l'industrie de traitement des aliments, comprenant un convoyeur de transport (11) sans fente dans le plan de transport pour transporter des produits individuels (12) dans la direction de transport T d'une extrémité d'entrée à une extrémité de sortie, une unité à rayons X (13) avec au moins une source de rayons X (14) et au moins une caméra à rayons X (15) pour saisir des informations spécifiques aux produits, la source de rayons X (14) et la caméra à rayons X (15) étant associées au convoyeur de transport (11) de telle sorte que les produits (12) peuvent être guidés entre la source de rayons X (14) et la caméra à rayons X (15), ainsi qu'une unité de commande (16, 19) qui est reliée à l'unité de rayons X (13) et qui est configurée et adaptée pour recevoir et évaluer les informations spécifiques aux produits saisies par l'unité de rayons X (13), qui forment un premier ensemble de données, au moins une caméra optique (17, 20) étant associée au même convoyeur de transport (11) entre son extrémité d'entrée et son extrémité de sortie, au moyen de laquelle, en plus de l'unité à rayons X (13), des informations spécifiques aux produits peuvent être saisies sur les produits (12) transportés sur le convoyeur de transport (11), la caméra optique (17) étant reliée à une unité de commande (18, 19) qui est configurée et adaptée pour recevoir et évaluer les informations spécifiques aux produits saisies par la caméra optique (17, 20), qui forment un deuxième ensemble de données, **caractérisé en ce que** l'unité à rayons X (13) et chaque caméra optique (17, 20) ainsi que le convoyeur de transport (11) sont raccordés à une unité de commande (19) pour la réception et l'évaluation des ensembles de données, l'unité de commande (19) étant équipée d'au moins un processeur (28) qui est configuré pour intégrer/implémenter au moins des parties des informations spécifiques aux produits sur un produit (12) saisies par l'unité à rayons X (13) dans l'image optique de la caméra optique (17, 20) concernant le même produit (12) pour former une image de transfert optique qui forme un troisième ensemble de données.

2. Dispositif (10) selon la revendication 1, **caractérisé en ce qu'**au moins une caméra optique (17, 20) est disposée au-dessus du convoyeur de transport (11).

3. Dispositif (10) selon la revendication 1 ou 2, **caractérisé en ce qu'**au moins deux caméras optiques (17, 20) sont associées au convoyeur de transport (11), qui sont équipées d'un procédé d'imagerie différent.

4. Dispositif (10) selon une ou plusieurs des revendications 1 à 3, **caractérisé en ce qu'**au moins une caméra optique (17, 20) est une caméra multi ou hyper spectrale.

5. Dispositif (10) selon une ou plusieurs des revendications 1 à 4, **caractérisé en ce qu'**au moins une caméra optique (17, 20) est configurée comme une caméra à niveaux de gris et/ou une caméra RVB et/ou une caméra thermique et/ou une caméra UV.

6. Dispositif (10) selon une ou plusieurs des revendications 1 à 5, **caractérisé en ce que** le convoyeur de transport (11) est une bande de transport de rayons X (22) à faible structure, entraînée en rotation et située sur la surface de transport T_{F} , qui est fabriquée en matière plastique, avec une bande de transport (23) comme brin supérieur et une bande de retour (24) comme brin inférieur.

7. Dispositif (10) selon une ou plusieurs des revendications 1 à 6, **caractérisé en ce que** le convoyeur de transport (11) est raccordé à une unité de commande (19) qui est configurée et adaptée pour recevoir et évaluer des données de mouvement du convoyeur de transport (11).

8. Dispositif (10) selon la revendication 7, **caractérisé en ce que** la source de rayons X (14) est disposée au-dessus de la bande de transport (23) et la caméra à rayons X (15) entre la bande de transport (23) et la bande de retour (24).

9. Dispositif (10) selon une ou plusieurs des revendications 1 à 8, **caractérisé en ce que** l'unité de commande (19) est configurée avec le processeur (28) pour déterminer des chemins de coupe à partir des informations spécifiques aux produits saisies par la caméra optique (17, 20) et à partir des informations spécifiques aux produits saisies par l'unité à rayons X (13), les chemins de coupe étant établis à partir des informations spécifiques aux produits sur un produit (12) saisies par l'unité à rayons X (13) directement sur l'image optique de la caméra optique (17, 20) vers le même produit (12), de telle sorte que l'unité de commande (19) met à disposition une image optique d'une caméra optique (17, 20), à savoir l'image de transfert, pour le traitement ultérieur.

10. Système (29) configuré et adapté pour traiter des produits (12) de l'industrie de traitement des aliments, comprenant un dispositif (10) configuré et adapté pour saisir et évaluer des informations spécifiques aux produits sur les produits (12), ainsi qu'un poste de traitement (30) disposé en aval du dispositif (10) dans la direction de transport T des produits (12), qui comprend un convoyeur de transport (31) pour transporter les produits (12) dans la direction de transport T d'une extrémité d'entrée à une extrémité de sortie, au moins une caméra optique (32) au moyen de laquelle des informations spécifiques aux produits peuvent être saisies sur les produits (12) transportés sur le convoyeur de transport (31) du poste de traitement (30), la caméra optique (32) étant reliée à une unité de commande (34), qui est configurée et adaptée pour recevoir et évaluer les informations spécifiques aux produits saisies par la caméra optique (34), ainsi qu'une unité de séparation (33) qui est configurée et adaptée pour couper et éliminer des zones indésirables des produits (12) et/ou pour diviser les produits (12) en portions, l'unité de séparation (33) étant reliée à une unité de commande (34) pour commander l'unité de séparation (33) sur la base d'informations spécifiques aux produits saisies et évaluées au préalable, **caractérisé en ce que** le dispositif (10), configuré et adapté pour saisir et évaluer des informations spécifiques aux produits sur les produits (12), est configuré selon l'une des revendications 1 à 9.

11. Système (29) selon la revendication 10, **caractérisé en ce que** toutes les unités de commande (34) du système (29) sont en liaison fonctionnelle les unes avec les autres et au moins une unité de commande (34) comprend un processeur (43) configuré pour superposer des images optiques des caméras optiques (17, 20, 32) du dispositif (10) d'une part et du poste de traitement (30) d'autre part, de telle sorte que l'identification des produits (12) ainsi que la mise en correspondance des positions relatives de chaque produit (12) sur les deux convoyeurs de transport (11, 31) du dispositif (10) d'une part et du poste de traitement (30) d'autre part s'effectuent exclusivement à partir d'images optiques de caméras optiques (17, 20, 32).

12. Système (29) selon la revendication 10 ou 11, **caractérisé en ce que** le dispositif (10) et le poste de traitement (30) sont connectés à une unité de commande (34) comprenant un processeur (43) configuré pour superposer une image optique de transfert, constituée d'une image optique d'une caméra optique (17, 20) du dispositif (10), enrichie de données spécifiques aux produits saisies par l'unité à rayons X (13) du dispositif (10), et une image optique de la caméra optique (32) du poste de traitement (30), de telle sorte que l'unité de séparation (33) peut être commandée au moyen de l'unité de commande (34) pour chaque produit (12) sur la base de chemins de coupe déterminés individuellement.

13. Système (29) selon une ou plusieurs des revendications 10 à 12, **caractérisé en ce que** l'unité de séparation (33) comprend une unité à jet d'eau (49) avec au moins une tuyère (50).

14. Système (29) selon une ou plusieurs des revendications 10 à 13, **caractérisé en ce qu'**au moins une caméra optique (17, 32) du dispositif (10) d'une part et de la station de traitement (30) d'autre part est équipée du même procédé d'imagerie.

15. Système (29) selon une ou plusieurs des revendications 10 à 14, **caractérisé en ce qu'**au moins une unité de commande (34) comprend au moins une mémoire (44), au moins un dispositif d'entrée (45) ainsi qu'au moins un dispositif de sortie (46).

16. Procédé de traitement de produits (12) de l'industrie de traitement des aliments, comprenant les étapes consistant à :
- transporter les produits (12) au moyen d'un premier convoyeur de transport (11) d'une extrémité d'entrée à une extrémité de sortie dans la direction de transport T,
- saisir des informations spécifiques aux produits pour chaque produit (12) sur le premier convoyeur de transport (11) au moyen d'une unité à rayons X (13), les informations saisies, qui forment un premier ensemble de données, étant reçues par une unité de commande (16, 19) et exploitées par celle-ci afin de déterminer des chemins de coupe pour découper des zones indésirables et/ou pour diviser en portions,
- transférer les produits du premier convoyeur de transport (11) à un convoyeur de transport suivant (31), au moyen duquel les produits (12) sont transportés dans la direction de transport T d'une extrémité d'entrée à une extrémité de sortie,
- saisir des informations spécifiques aux produits pour chaque produit (12) sur le convoyeur de transport suivant (31) au moyen d'une caméra optique (32), les informations saisies, qui forment un quatrième ensemble de données, étant reçues par une unité de commande (34) et exploitées par celle-ci afin d'identifier chaque produit (12),
- comparer et évaluer des ensembles de données reçus et exploités au moyen d'une unité de commande (34), afin de calculer les positions de produit relatives de chaque produit (12) sur les deux convoyeurs de transport (11, 31), et
- couper les produits (12) le long des chemins de coupe précédemment déterminés et associés au produit respectif au moyen d'une unité de séparation (33), l'unité de séparation (33) étant commandée par une unité de commande (34),
des informations spécifiques aux produits peuvent être saisies sur chaque produits (12) au moyen d'une caméra optique (17, 20) sur le premier convoyeur de transport (11), en plus des informations spécifiques aux produits saisies de l'unité à rayons X (13), les informations saisies par la caméra optique (17, 20) associée au premier convoyeur de transport (11), qui forment un deuxième ensemble de données, étant reçues et exploitées par une unité de commande (18, 19, 34), **caractérisé en ce que** le premier ensemble de données, qui contient les chemins de coupe générés par l'unité à rayons X (13), est intégré/implémenté dans le deuxième ensemble de données, à savoir dans l'image optique générée par la caméra optique (17, 20) associée au premier convoyeur de transport (11), de sorte qu'une image de transfert optique est transmise à l'unité de commande (19, 34) en tant que troisième ensemble de données pour la comparaison et l'évaluation, et **en ce que** l'identification des produits (12) et la comparaison et l'évaluation des ensembles de données s'effectuent sur la base du troisième et du quatrième ensemble de données.

17. Procédé selon la revendication 16, **caractérisé en ce que** les positions des zones indésirables générées par l'unité à rayons X (13) et/ou une autre caméra optique (17, 20) sont intégrées/implémentées sous forme de données vectorielles, de données de pixels ou similaires dans l'image optique de la caméra optique (17, 20) associée au premier convoyeur de transport (11, et **en ce que** la comparaison ("matching") et l'évaluation sont effectuées sur la base de l'image optique de la caméra optique (17, 20) associée au premier convoyeur de transport (11) et de l'image optique de la caméra optique (32) associée au deuxième convoyeur de transport (31).

18. Procédé selon la revendication 16 ou 17, **caractérisé en ce que**, pour identifier les produits, chaque image optique prise par la caméra optique (32) associée au convoyeur de transport suivant (31) est comparée avec les images optiques de la caméra optique (17, 20) associée au premier convoyeur de transport (11), sauvegardées dans une mémoire (44) d'une unité de commande (34), et l'image présentant les plus grandes correspondances est sélectionnée.

19. Procédé selon une ou plusieurs des revendications 16 à 18, **caractérisé en ce qu'**il est mis en oeuvre avec un système (29) selon l'une des revendications 10 à 15.
